(19) Europäisches Patentamt
European Patent Office
Office européen des brevets

(11) Numéro de publication: **0 239 445 B1**

(12) # FASCICULE DE BREVET EUROPEEN

(45) Date de publication de fascicule du brevet: **13.05.92**

(51) Int. Cl.[5]: **C07D 211/70**, A61K 31/445

(21) Numéro de dépôt: **87400407.0**

(22) Date de dépôt: **24.02.87**

(54) **Dérivés du 1,2,5,6-tétrahydropyridin-3-carboxaldéhyde oxime, leur procédé de préparation, leur application comme médicaments et les compositions les renfermant.**

(30) Priorité: **27.02.86 IT 1956586**
**17.07.86 IT 2115786**

(43) Date de publication de la demande:
**30.09.87 Bulletin 87/40**

(45) Mention de la délivrance du brevet:
**13.05.92 Bulletin 92/20**

(84) Etats contractants désignés:
**AT BE CH DE ES FR GB GR IT LI LU NL SE**

(56) Documents cités:
**EP-A- 0 271 798**
**FR-A- 1 258 847**

**JOURNAL OF PHARMACEUTICAL SCIENCES, vol. 56, no. 9, septembre 1967, pages 1190-1192; J.N. WELLS et al.: "Synthesis of aldoxime analogs of arecoline as reactivators of organophosphorus inhibited cholinesterase"**

(73) Titulaire: **ROUSSEL-UCLAF**
**35, Boulevard des Invalides**
**F-75007 Paris(FR)**

(72) Inventeur: **Galliani, Giulio**
**Via U. Biancamano**
**I-20052 Monza (MI)(IT)**
Inventeur: **Barzaghi, Fernando**
**Via Monteverdi 21**
**I-20052 Monza (MI)(IT)**
Inventeur: **Butti, Alina**
**Via Palmanova 189**
**I-20100 Milano(IT)**
Inventeur: **Bonetti, Carla**
**Via Beccalino**
**I-Fontanella (BG)(IT)**
Inventeur: **Toja, Emilio**
**Via Plezzo 80**
**I-20100 Milano(IT)**

(74) Mandataire: **Tonnellier, Marie-José et al**
**Département des Brevets ROUSSEL UCLAF**
**B.P. no 9**
**F-93230 Romainville(FR)**

**EP 0 239 445 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

**EUROPEAN JOURNAL OF MEDICINAL CHEMISTRY, CHIMICA THERAPEUTICA, vol. 9, no. 1, janvier/février 1974, pages 101-107; P. VERGNON et al.: "Dérivés vinylcyclopropaniques II - Pharmacologie de l'oxime de l'acétyl-1 vinyl-2 cyclopropane"**

**CHEMICAL ABSTRACTS, vol. 90, no. 21, 21 mai 1979, page 585, résumé no. 168416x, Columbus, Ohio, US; E.K. ORLOVA et al.: "Synthesis and neurotropic properties of some alpha-aminobenzyl piperidines"**

## Description

L'invention concerne de nouveaux dérivés du 1,2,5,6-tétrahydropyridin-3-carboxaldéhyde oxime, leur procédé de préparation, leur application comme médicaments et les compositions les renfermant.

On connaissait déjà des dérivés de la 1,2,5,6-tétrahydropyridin-3-carboxaldéhyde oxime, comme la 1,2,5,6-tétrahydropyridin-3-carboxaldéhyde oxime (Chem. Ber. 40, 4685, 1907) ou encore la 1-méthyl 1,2,5,6-tétrahydropyridin-3-carboxaldéhyde oxime (Chem. Ber. 40, 4712, 1907) ou encore la 1-éthyl 1,2,5,6-tétrahydropyridin-3-carboxaldéhyde oxime (Chem. Ber. 38, 4161, 1905), c'est-à-dire des composés répondant à la formule générale :

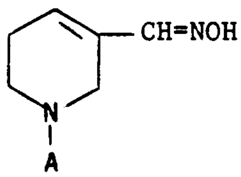

A=H, alcoyle

Certains de ces produits avaient même été étudiés en pharmacologie (cf. Journal of Pharmaceutical Sciences, 1190 Vol. 56, 1967 et leur intérêt avait été jugé très faible, les produits étant beaucoup moins actifs que l'arécoline.

On vient de découvrir que, d'une façon inattendue, certains produits de structure chimique voisine présentent de très intéressantes propriétés pharmacologiques, très supérieures à celle de l'arécoline comme le montrent les résultats des tests pharmacologiques décrits ci-après.

L'invention a pour objet les composés de formule (I) :

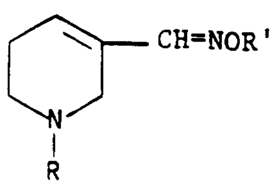

(I)

dans laquelle R représente un atome d'hydrogène, un radical alcoyle linéaire, ramifié ou cyclique, saturé ou insaturé, renfermant jusqu'à 8 atomes de carbone, éventuellement substitué par un radical méthoxycarbonyle, éthoxycarbonyle, propoxycarbonyle linéaire ou ramifié, ou butoxycarbonyle linéaire ou ramifié, R représente un radical aralcoyle renfermant jusqu'à 10 atomes de carbone et R' représente un radical alcoyle, linéaire ou ramifié, saturé ou insaturé, renfermant jusqu'à 8 atomes de carbone, un radical $-COalc_1$ ou un radical $-(CH_2)_2N(alc_2)_2$, $alc_1$ et $alc_2$ représentant un radical alcoyle renfermant jusqu'à 8 atomes de carbone, ainsi que leurs sels d'addition avec les acides.

Parmi les sels d'addition avec les acides, on peut citer ceux formés avec les acides minéraux, tels que les acides chlorhydrique, bromhydrique, sulfurique ou phosphorique ou avec les acides organiques comme l'acide formique, acétique, propionique, benzoïque, maléïque, fumarique, succinique, tartrique, citrique, oxalique, glyoxylique, aspartique, alcanesulfoniques, tels que les acides méthane ou éthane sulfoniques, arylsulfoniques tels que les acides benzène ou paratoluènesulfoniques.

Lorsque R ou R' représente un radical alcoyle saturé, linéaire ou ramifié, il s'agit de préférence d'un radical méthyle, éthyle, n-propyle, isopropyle, n-butyle, sec-butyle, isobutyle, n-pentyle, n-hexyle, tert-butyle, tert-pentyle, néopentyle ou n-hexyle.

Lorsque R ou R' représente un radical alcoyle insaturé, il s'agit de préférence q'un radical éthylénique comme, par exemple, d'un radical vinyle, allyle, 1,1-diméthylallyle, but-2-ényl, ou d'un radical acétylénique comme, par exemple, le radical éthynyle ou propynyle.

Lorsque R représente un radical alcoyle cyclique, il s'agit de préférence q'un radical cyclopropyle, cyclobutyle, cyclopentyle, cyclonexyle, cyclopropylméthyle, cyclobutylméthyle, cyclopentylméthyle ou cyclohexylméthyle.

Lorsque R représente un radical aralcoyle, il s'agit de préférence d'un radical benzyle ou phénétyle.

$Alc_1$ et $alc_2$ représentent de préférence un radical méthyle, éthyle, n-propyle, isopropyle, n-butyle, sec-butyle ou isobutyle.

L'invention a plus spécialement pour objet les composés de formule (I) dans lesquels R représente un atome d'hydrogène ainsi que leurs sels d'addition avec les acides.

L'invention a également plus spécialement pour objet les composés de formule (I) dans lesquels R représente un radical alcoyle saturé ou insaturé renfermant de 1 à 4 atomes de carbone et notamment un radical méthyle, éthyle, propyle ou allyle, ainsi que leurs sels d'addition avec les acides.

Parmi les composés préférés de l'invention, on peut citer les composés dans lesquels R' représente un radical méthyle ainsi que leurs sels d'addition avec les acides.

L'invention a tout particulièrement pour objet les composés dont la préparation est indiquée de façon détaillée dans la partie expérimentale. Parmi ces composés, on peut citer comme objet préféré de l'invention, les composés décrits aux exemples 1, 3, 7, 8 et 10.

Les composés de l'invention présentent de très intéressantes propriétés pharmacologiques et notamment une activité cholinomimétique par voie orale importante de longue durée d'action.

Il est bien connu que les troubles de l'apprentissage et de la mémoire chez les personnes âgées sont surtout reliés à un déficit du système cholinergique central, en particulier dans la demeure sénile et la maladie d'Alzheimer.

Il est donc évident que les produits ayant une action cholinergique centrale puissent être employés dans le traitement thérapeutique de ces maladies (Bartus, R.I. Science 217, 408, 1982).

Il a été démontré que l'arécoline injectée par voie intraveineuse a un effet positif sur de patients ayant un déficit de la mémoire (Sitaram N. et al. Science 201, 274, 1978)(Christie J.E. et al Brit. J. Psychiatry 138, 46, 1981).

Une limitation à l'emploi thérapeutique de l'arécoline est reliée au fait que ce produit a une très faible activité par voie orale et une courte durée d'action.

Les produits, objet de l'invention, ont démontré, après administration par voie orale, une activité cholinomimétique centrale jusqu'à 1500 fois supérieure à celle de l'arécoline et avec une plus longue durée d'action.

L'invention a donc pour objet les produits de l'invention en tant que médicaments, utiles notamment dans le traitement de la maladie d'Alzheimer ou de la démence sénile et également dans le traitement des troubles de la mémoire.

L'invention a plus particulièrement pour objet en tant que médicaments, les composés des exemples 1, 3, 7, 8 et 10.

La posologie usuelle est variable selon l'affection en cause, le sujet traité et la voie d'administration, elle peut être comprise entre 1 mg et 100 mg/jour, par exemple, entre 1 et 15 mg/jour en une ou plusieurs prises pour le produit de l'exemple 3 administré par voie orale.

La présente invention a également pour objet les compositions phrarmaceutiques renfermant comme principe actif au moins un produit de formule (I). Les compositions pharmaceutiques de l'invention peuvent être solides ou liquides et se présenter sous les formes pharmaceutiques couramment utilisées en médecine humaine, comme, par exemple, les comprimés simples ou dragéifiés, les gélules, les granulés, les suppositoires, les préparations injectables ; elles sont préparées selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention a également pour objet un procédé de préparation des composés de formule (I), caractérisé en ce que l'on soumet un composé de formule (II) :

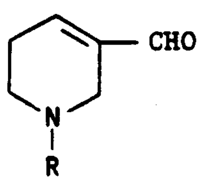

(II)

ou l'un de ses sels,

dans laquelle R conserve la même signification que précédemment, à l'action d'un composé de formule (III) :

$NH_2OR'_1$ (III)

ou l'un de ses sels,

dans laquelle $R'_1$ représente un atome d'hydrogène ou un radical alcoyle linéaire ou ramifié, saturé ou insaturé, renfermant jusqu'à 8 atomes de carbone, pour obtenir le composé de formule ($I_A$) correspondant :

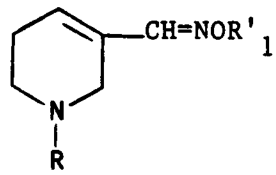

($I_A$)

que l'on salifie le cas échéant, ou que l'on soumet, dans le cas où $R'_1$ représente un atome d'hydrogène, à l'action d'un composé de formule (IV) :

$R'_2$Hal (IV)

$R'_2$ représentant un radical COalc$_1$ ou un radical $-(CH_2)_2N-(alc_2)_2$, alc$_1$ et alc$_2$ étant définis comme précédemment et Hal représentant un atome d'halogène, pour obtenir le composé de formule ($I_B$) correspondant :

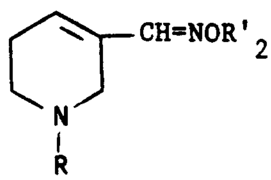

($I_B$)

que l'on salifie le cas échéant, composé de formule ($I_A$) dans lequel $R'_1$ ne représente pas un atome d'hydrogène ou composé de formule ($I_B$) que l'on soumet, le cas échéant, dans le cas où R représente un atome d'hydrogène, à l'action d'un composé de formule (VI) :

$R_1$Hal (VI)

dans lequel Hal représente un atome d'halogène et $R_1$ représente un radical alcoyle, linéaire, ramifié ou cyclique, saturé ou insaturé, renfermant jusqu'à 8 atomes de carbone, éventuellement substitué par un radical carboxy estérifié ou $R_1$ représente un radical aralcoyle renfermant jusqu'à 10 atomes de carbone, pour obtenir un composé de formule ($I_C$) :

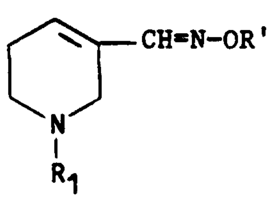

($I_C$)

dans lequel $R_1$ et $R'$ ont la signification indiquée précédemment, que le cas échéant, l'on salifie, ou que l'on soumet, dans le cas où
$R_1$ représente un radical alcoyle substitué par un radical carboxy estérifié, à l'action d'un agent d'hydrolyse pour obtenir le composé correspondant comportant un radical carboxy libre, composé que, le cas échéant, l'on salifie.

Dans un mode de réalisation préférée du procédé de l'invention :

- le composé de formule (II) et le composé de formule (III) sont utilisés sous forme de chlorhydrate,
- dans les composés $R'_2$Hal et $R_1$Hal, Hal représente un atome de chlore ou de brome,
- l'agent d'hydrolyse est l'acide paratoluène sulfonique.

L'invention a également pour objet une variante du procédé précédent caractérisé en ce que l'on soumet un composé de formule (V) :

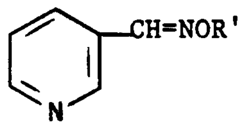

(V)

dans laquelle R′ conserve la même signification que précédemment, à l'action d'un composé de formule (VI) tel que défini précédemment, pour obtenir le composé de formule (VII) :

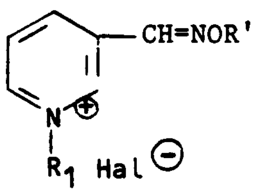

(VII)

que l'on soumet à l'action d'un agent d'hydrogénation, pour obtenir le composé de formule ($I_C$) correspondant :

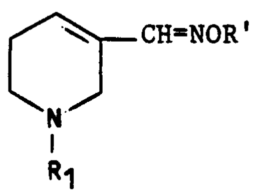

($I_C$)

dans lequel $R_1$ et R′ ont la signification indiquée précédemment que, le cas échéant, l'on salifie ou l'on soumet, dans le cas où $R_1$ représente un radical alcoyle substitué par un radical carboxy estérifié, à l'action d'un agent d'hydrolyse pour obtenir le composé correspondant comportant un radical carboxy libre, composé que le cas échéant, l'on salifie.

Dans un mode de réalisation préféré, l'agent d'hydrogénation utilisé est l'hydroborure de sodium et l'agent d'hydrolyse est l'acide paratoluène sulfonique.

Les composés de formule (II) sont des produits connus d'une façon générale, ils peuvent être préparés selon le procédé décrit dans Chem. Ber. 40, 4685, 1907.

Les produits de formule ($I_A$) dans lesquels $R'_1$ représente un atome d'hydrogène sont des produits connus qui peuvent être préparés selon le procédé décrit dans Chem. Ber. 40, 4712, 1907.

Les composés de formule (V) sont également des produits connus d'une façon générale qui peuvent être préparés selon le procédé décrit dans J. Het. Chem. 1979, 1459.

Les exemples suivants illustrent l'invention sans toutefois la limiter.

**Exemple 1 : Chlorhydrate de 1-méthyl 1,2,5,6,tétrahydropyridin-3-carboxaldéhyde 0-méthyloxime.**

On ajoute 1,42 g de chlorhydrate de 1-méthyl hydroxylamine dans une solution comprenant 2,74 g de chlorhydrate de 1-méthyl 1,2,5,6-tétrahydro pyridin 3-carboxaldéhyde dans 10 cm3 d'eau et on agite 2 heures à température ambiante. On évapore le solvant, reprend le résidu à l'acétone, filtre le produit et le recristallise dans l'éthanol. On obtient 1,75 g de produit cristallisé. F = 228¤C (Décomposition).
Analyse : $C_8H_{14}N_2O$, HCl : 190,681.
Calculé : C% 50,39 H% 7,93 N% 14,69
Trouvé : 50,57 7,94 14,56.

**Exemple 2 : Chlorhydrate de 1-méthyl 1,2,5,6-tétrahydropyridin-3-carboxaldéhyde 0-éthyloxime.**

On ajoute 2,42 g de chlorhydrate de O-éthyl hydroxylamine dans une solution comprenant 4 g de chlorhydrate de 1-méthyl 1,2,5,6-tétrahydro pyridin 3-carboxaldéhyde (Chem. Ber. 40, 4712, 1907) dans 15 cm3 d'eau. On agite le mélange pendant 1 heure à température ambiante. On concentre à sec et cristallise le produit dans l'éthanol. On obtient 3,1 g de produit attendu. F = 197¤C (décomposition).
Analyse : $C_9H_{16}N_2O$, HCl : 204,708.
Calculé : C% 52,80 H% 8,37 N% 13,68
Trouvé : 52,61 8,47 13,47.

**Exemple 3 : Chlorhydrate de 1,2,5,6-tétrahydropyrìdin-3-carboxaldéhyde 0-méthyloxime.**

On dissout 5 g de chlorhydrate de 1,2,5,6-tétrahydropyridin 3-carboxaldéhyde (Chem. Ber. 40, 4685, 1907) dans 30 cm3 d'eau, ajoute 2,85 g de chlorhydrate de 0-hydroxylamine et agite 1 heure à température ambiante. On évapore à sec, cristallise le résidu dans l'éthanol et obtient 4,8 g de produit attendu. F = 208¤C (décomposition).
Analyse : $C_7H_{12}N_2O$, HCl : 176,654.
Calculé : C% 47,59 H% 7,42 N% 15,86
Trouvé : 47,42 7,38 15,63.

**Exemple 4 : Chlorhydrate de 0-isopropyl N-méthyl 1,2,5,6-tétrahydro pyridin-3-carboxaldéhyde oxime.**

On dissout 1,24 g de chlorhydrate de 1-méthyl 1,2,5,6-tétrahydro pyridin 3-carboxaldéhyde dans 10 cm3 d'eau, ajoute 0,86 g de chlorhydrate de 0-isopropyl hydroxylamine et agite 1 heure à température ambiante. On évapore le solvant et recristallise le résidu dans l'éthanol. On obtient 1 g de produit attendu. F = 234¤C (décomposition).
Analyse : $C_{10}H_{18}N_2O$, HCl : 218,735.
Calculé : C% 54,91 H% 8,75 N% 12,81
Trouvé : 55,04 8,84 12,73.

**Exemple 5 : 1-méthyl 1,2,5,6-tétrahydropyridin-3-carboxaldéhyde 0-acétyloxime.**

On dissout 2 g de 1-méthyl 1,2,5,6-tétrahydropyridin 3-carboxaldéhyde oxime (Chem. Ber. 40, 4712, 1907) dans 20 cm3 de tétrahydrofuranne, ajoute 1,44 g de triéthylamine et 1,12 g de chlorure d'cétyle et agite 1 heure à température ambiante. On lave le milieu réactionnel à l'eau, puis avec une solution aqueuse de bicarbonate de sodium, sépare la phase organique, la sèche et élimine le solbant. On distille le résidu sous une pression de 0,05 mm de mercure et rècupére la fraction dont le point d'ébullition est compris entre 170 et 175¤C. On obtient 2,1 g de produit attendu.
Analyse : $C_9H_{14}N_2O_2$, HCl : 182,227.
Calculé : C% 59,32 H% 7,74 N% 15,37
Trouvé : 59,54 7,72 15,49.

**Exemple 6 : 3-[2-(N,N-diméthylaminoéthoxyimino) méthyl] 1-méthyl 1,2,5,6-tétrahydropyridine.**

Dans une solution d'éthylate de sodium (obtenue à partir de 0,66 g de sodium dans 40 cm3 d'éthanol), on ajoute 2 g de 1-méthyl 1,2,5,6-tétrahydropyridin 3-carboxaldéhyde oxime (Chem. Ber. 40, 4712, 1907) et 2,06 g de chlorhydrate du chlorure de béta-diméthylaminoéthyle. On chauffe 2 heures à température d'ébullition, refroidit et élimine le solvant. On reprend le résidu dans la soude 2 N et extrait à l'acétate d'éthyle. On sépare la phase organique, sèche et évapore le solvant. On distille le résidu sous une pression de 0,02 mm de mercure et récupère la fraction dont le point d'ébullition est compris entre 100 et 105¤C. On obtient 2,3 g de produit attendu.
Analyse : $C_{11}H_{21}N_3O$, HCl : 211,312.
Calculé : C% 62,52 H% 10,01 N% 19,88
Trouvé : 62,21 9,84 19,84.

**Exemple 7 : Chlorhydrate de 1-éthyl 1,2,5,6-tétrahydro 3-carboxaldéhyde 0-méthyloxime.**

**Stade A :** Iodure de 1-éthyl 3-(méthoxyiminométhyl) pyridine.

On chauffe pendant 5 heures à la température d'ébullition une solution comprenant 10,6 g de 3-pyridin carboxaldéhyde 0-méthyloxime (J. Het. Chem. (1979) 1459) et 12 g de iodoéthane dans 100 cm3 d'acétone. On ajoute de nouveau 7,8 g d'iodoéthane et poursuit le chauffage pendant 4 heures. On refroidit le milieu réactionnel, élimine le solvant. Le résidu huileux se solidifie au cours du refroidissement. On obtient 16 g de produit attendu. F = 118¤C (décomposition).

**Stade B :** Chlorhydrate de 1-éthyl 1,2,5,6-tétrahydro 3-carboxaldéhyde 0-méthyloxime.

On ajoute 2,5 g de borohydrure de sodium dans une solution comprenant 10 g d'iodure de 1-éthyl 3-(méthoxyiminométhyl) pyridine dans 100 cm3 de méthanol en maintenant la température à 20-22¤C. On agite pendant 1 heure, élimine le solvant, reprend le résidu par de l'acide chlorhydrique 2N, alcalinise avec du bicarbonate de sodium et extrait à l'acétate d'éthyle. On sépare la phase organique, la sèche et élimine le solvant. On reprend le résidu huileux dans l'éther, filtre, fait barboter de l'acide chlorhydrique gazeux et sépare le chlorhydrate obtenu par filtration. Après recristallisation dans l'éthanol, on obtient 1,5 g de produit cristallisé attendu. F = 220¤C (décomposition).
Analyse : $C_9H_{16}N_2O$, HCl : 204,708.
Calculé : C% 52,80 H% 8,37 N% 13,68
Trouvé : 52,65 8,34 13,42.

**Exemple 8 : 1-propyl 1,2,5,6-tétrahydropyridin 3-carboxaldéhyde 0-méthyl oxime.**

On ajoute 1,74 g de triéthylamine et 1,05 g de 1-bromopropane dans une solution comprenant 1,5 g de chlorhydrate de 1,2,5,6-tétrahydropyridin 3-carboxaldéhyde 0-méthyloxime dans 15 cm3 de diméthylformamide. On chauffe le milieu réactionnel à 70¤C pendant 2 heures, refroidit, concentre à sec, reprend le résidu dans une solution aqueuse de bicarbonate de sodium et extrait à l'acétate d'éthyle. On sépare la phase organique, la sèche et évapore le solvant. On chromatographie le résidu sur silice (éluant : acétone-acétate d'éthyl 1-1). Après élimination du solvant, on obtient une huile que l'on traite avec de l'acide chlorhydrique gazeux dans l'éther éthylique. On recristallise le chlorhydrate obtenu dans l'éthanol et obtient 0,9 g de produit attendu. F = 220¤C (décomposition).
Analyse : $C_{10}H_{18}N_2O$, HCl
Calculé : C% 54,91 H% 8,75 N% 12,81
Trouvé : 55,04 8,91 12,74.

**Exemple 9 : Chlorhydrate de 1-butyl 1,2,5,6-tétrahydropyridin 3-carboxaldéhyde 0-méthyloxime.**

On ajoute 1,26 g de triéthylamine et 0,85 g de 1-bromo butane à un mélange comprenant 1,1 g de chlorhydrate de 1,2,5,6-tétrahydropyridin 3-carboxaldéhyde 0-méthyloxime dans 15 cm3 de diméthylformamide. On agite 2 heures à température ambiante, concentre à sec, reprend le résidu dans une solution aqueuse de carbonate de potassium et extrait à l'acétate d'éthyle. On sépare la phase organique, la sèche et évapore le solvant. On chromatographie le résidu sur silice en éluant avec un mélange chloroforme-éthanol (7-3). Après élimination du solvant, on traite le résidu avec de l'acide chlorhydrique gazeux dans l'éther éthylique, filtre et recristallise le chlorhydrate dans un mélange éthanol-éther éthylique. On obtient 0,85 g de produit attendu. F = 186¤C (décomposition).
Analyse : $C_{11}H_{20}N_2O$, HCl
Calculé : C% 56,76 H% 9,09 N% 12,04
Trouvé : 56,61 8,93 11,92.

**Exemple 10 : Chlorhydrate de 1-allyl 1,2,5,6-tétrahydropyridin-3-carboxaldéhyde 0-méthyloxime.**

On ajoute 1,38 g de triéthylamine et 0,825 g de bromure d'allyle dans une solution comprenant 1,2 g de chlorhydrate de 1,2,5,6-tétrahydropyridin 3-carboxaldéhyde 0-méthyloxime dans 15 cm3 de diméthylformamide. On agite le mélange réactionnel pendant 2 heures à température ambiante, concentre à sec, reprend le résidu dans une solution aqueuse de carbonate de potassium et extrait à l'acétate d'éthyle. On sépare la phase organique, la sèche et évapore le solvant. On dissout le résidu dans l'éther éthylique, filtre et salifie

avec de l'acide chlorhydrique gazeux. On recristallise le chlorhydrate obtenu dans l'éthanol et obtient 1,2 g de produit attendu. F = 220¤C (décomposition).
Analyse : $C_{10}H_{16}N_2O$, HCl : 190,681.
Calculé : C% 55,42 H% 7,91 N% 12,93
Trouvé : 55,02 7,72 12,74.

**Exemple 11 : Chlorhydrate de 1-pentyl 1,2,5,6-tétrahydropyridin 3-carboxaldéhyde 0-méthyloxime.**

On ajoute 1,72 g de triéthylamine et 1,28 g de 1-bromo pentane dans un mélange comprenant 1,5 g de chlorhydrate de 1,2,5,6-tétrahydropyridin 3-carboxaldéhyde 0-méthyloxime dans 20 cm3 de diméthylformamide. On agire pendant 3 heures à température ambiante, concentre à sec, reprend le résidu dans l'eau et extrait à l'acétate d'éthyle. On sépare la phase organique, la sèche et élimine le solvant. On reprend le résidu dans l'éther, salifie avec de l'acide chlorhydrique gazeux, filtre et recristallise le chlorhydrate obtenu dans un mélange méthanol-éther. On obtient 1,1 g de produit attendu. F = 185¤C (décomposition).
Analyse : $C_{12}H_{22}N_2O$, HCl : 190,681.
Calculé : C% 58,40 H% 9,39 N% 11,35
Trouvé : 58,27 9,48 11,19.

**Exemple 12 : Chlorhydrate de 1-méthyl 1,2,5,6-tétrahydropyridin 3-carboxaldéhyde 0-propargyloxime.**

On ajoute 1,23 g de chlorhydrate de 0-propargyl hydroxylamine dans une solution comprenant 1,84 g de chlorhydrate de 1-méthyl 1,2,5,6-tétrahydro pyridin 3-carboxaldéhyde dans 15 cm3 d'eau. On agite 1 heure à température ambiante, concentre à sec et cristallise le résidu dans l'éthanol. On obtient 2,2 g de produit attendu. F = 157¤C (décomposition).
Analyse : $C_{10}H_{14}N_2O$, HCl : 190,681.
Calculé : C% 55,94 H% 7,04 N% 13,05
Trouvé : 55,81 6,95 13,11.

**Exemple 13 : Chlorhydrate de 1-benzyl 1,2,5,6-tétrahydropyridin-3-carboxaldéhyde 0-méthyloxime.**

**Stade A :** Bromure de 1-benzyl 3-carboxaldéhyde 0-méthyloxime pyridinium.

On dissout 22 g de 3-aldoxime pyridine 0-méthyléther (J.Hel. Chem. 1979, p. 1459) dans l'éthanol anhydre, ajoute 40,2 g de bromure de benzyle et chauffe 12 heures au reflux. On élimine le solvant sous pression réduite, reprend le résidu avec un mélange éthanol-éther anhydre, filtre et obtient 39,5 g de produit attendu. F = 103-106¤C.

**Stade B :** Chlorhydrate de 1-benzyl 1,2,5,6-tétrahydropyridin-3-carboxaldéhyde 0-méthyloxime.

On dissout 30 g de produit obtenu ci-dessus dans 250 cm3 de méthanol anhydre, refroidit à 5-10¤C et ajoute 4,8 g de borohydrure de sodium par petites fractions en maintenant la température à 5-10¤C. On agite 2 heures à température ambiante puis élimine le solvant à 40¤C sous pression réduite. On chromatographie le résidu sur silice, élue par un mélange acétate d'éthyle-toluène (6-4) et obtient 13 g de produit que l'on transforme (eb : 230¤C sous 0,05 mm Hg) en chlorhydrate. F = 261¤C (décomposition) après cristallisation dans l'éthanol 95%.
Analyse : $C_{14}H_{18}N_2O$, HCl : 266,775
Calculé : C% 63,03 H% 7,18 N% 10,50
Trouvé : 62,88 7,31 10,24

**Exemple 14 : Chlorhydrate de 1-cyclopropane 1,2,5,6-tétrahydropyridin-3-carboxaldéhyde 0-méthyloxime.**

On ajoute 0,9 g de chlorométhylcyclopropane à 2,8 g de 1,2,5,6-tétrahydro-3-carboxaldéhyde 0-méthyloxime et chauffe à 80¤C pendant 3 heures puis on ajoute 0,45 g de chlorométhylcyclopropane et maintient la température à 80¤C pendant 6 heures. On refroidit, délaie dans l'éther éthylique anhydre, filtre et chromatographie le filtrat sur silice en éluant à l'acétate d'éthyle. Après élimination du solvant, on obtient 1,4 g d'une huile qui distille à 130¤C sous 0,1 mm Hg. On acidifie avec de l'acide chlorhydrique gazeux

dans l'éther éthylique anhydre et cristallise dans un mélange éthanol-éther éthylique. F = 233¤C (décomposition).
Analyse : $C_{11}H_{18}N_2O$, HCl : 230,745
Calculé : C% 57,25 H% 8,30 N% 12,14
Trouvé : 57,03 8,17 11,96

**Préparation du 1,2,5,6-tétrahydropyridin-3-carboxaldéhyde 0-méthyloxime utilisé au départ de l'exemple 14.**

**Stade A :** 1-alpha-chloroéthoxycarbonyl 1,2,5,6-tétrahydropyridin-3-carboxaldéhyde 0-méthyloxime.

On refroidit à 0¤C une solution de 13,2 g de 1-benzyl 1,2,5,6-tétrahydropyridin-3-carboxaldéhyde 0-méthyloxime dans 120 cm3 de 1,2-dichloroéthane anhydre, ajoute 11,7 g de chloroformiate d'alpha-chloroéthyle et chauffe à reflux pendant 2 heures. On refroidit et filtre l'insoluble. On évapore à sec le filtrat, reprend le résidu avec de l'éther anhydre, délaie et filtre. On évapore le filtrat et obtient 19,8 g de produit que l'on utilise immédiatement pour la réaction suivante.

**Stade B :** 1,2,5,6-tétrahydropyridin-3-carboxaldéhyde 0-méthyloxime.

On dissout 19 g de produit obtenu ci-dessus dans 100 cm3 de méthanol anhydre et chauffe à 50¤C pendant 1 heure. On évapore à sec le solvant, reprend le résidu à l'éther éthylique anhydre, agite, filtre et obtient 8,4 g de produit attendu.

**Exemple 15 : Chlorhydrate de 1,1-diméthyléthoxycarbonylméthyl-1,2,5,6-tétrahydropyridin-3-carboxaldéhyde 0-méthyloxime.**

On dissout 4,5 g de 1,2,5,6-tétrahydropyridin-3-carboxaldéhyde 0-méthyloxime préparé à l'exemple 14 dans 30 cm3 de benzène anhydre, on ajoute 3,25 g de triéthylamine et lentement 6,3 g de bremoacétate de tributyle. Après 30 minutes de réaction, on filtre le chlorure de triéthylamine formé, évapore le benzène, distille à 130¤C sous 1 mm Hg et obtient 6 g de produit huileux. On prépare le chlorhydrate avec l'acide chlorhydrique gazeux dans l'éther éthylique anhydre et le recristallise dans un mélange éthanol-éther anhydre. F = 182¤C (décomposition).
Analyse : $C_{13}H_{22}N_2O_3$, HCl : 290,797
Calculé : C% 53,69 H% 7,97 N% 9,63
Trouvé : 53,87 8,03 9,81

**Exemple 16 : Chlorhydrate de 1-carboxyméthyl 1,2,5,6-tétrahydropyridin-3-carboxaldéhyde0-méthyloxime.**

On dissout 3 g de chlorhydrate de 1-(1,1-diméthyléthoxycarbonylméthyl) 1,2,5,6-tétrahydropyridin-3-carboxaldéhyde 0-méthyloxime dans 30 cm3 de toluène anhydre, ajoute 2,27 g d'acide paratoluène sulfonique et chauffe au reflux pendant 1 heure. On évapore à sec, reprend le résidu au 1,2-dichloroéthane, salifie avec de l'acide chlorhydrique gazeux et précipite à l'éther éthylique anhydre. On filtre et recristallise dans l'éthanol pour obtenir 1,8 g de produit attendu. F = 213¤C (décomposition).
Analyse : $C_9H_{14}N_2O_3$, HCl : 234,692
Calculé : C% 46,06 H% 6,44 N% 11,94
Trouvé : 45,92 6,27 11,91

**Exemple 17 : Chlorhydrate de 1-(but-2-ényl) 1,2,5,6-tétrahydropyridin-3-carboxaldéhyde 0-méthyloxime.**

On opère comme à l'exemple 15 dans du diméthylformamide avec le bromure de crotyle en agitant 1 heure à température ambiante. On reprend le résidu sec avec un peu d'eau et extrait à l'acétate d'éthyle. Le chlorhydrate obtenu fond à 215¤C (décomposition).
Analyse : $C_{11}H_{18}N_2O$, HCl : 230,745
Calculé : C% 57,26 H% 7,86 N% 12,14
Trouvé : 57,02 8,06 12,07

**Exemple 18 : Chlorhydrate de 1-(prop-2-yl) 1,2,5,6-tétrahydropyridin-3-carboxaldéhyde 0-méthyloxime.**

On chauffe au reflux pendant 1 heure, 3,2 g de 1,2,5,6-tétrahydropyridin-3-carboxaldéhyde 0-méthyloxime avec 1,41 g de 2-bromopropane. On alcalinise avec une solution aqueuse à 10% de carbonate de potassium, extrait à l'acétate d'éthyle puis évapore à sec. On chromatographie sur silice le résidu, élue par un mélange méthanol-chloroforme (2-8). On obtient 1,2 g d'une huile qui distille à 110¤C sous 0,08 mm Hg. On acidifie avec l'acide chlorhydrique gazeux dans l'éther. Après recristallisation dans l'alcool isopropylique-éther éthylique, le chlorhydrate fond à 210¤C (décomposition).

Analyse : $C_{10}H_{18}N_2O$, HCl : 218,728
Calculé : C% 54,91 H% 8,76 N% 12,81
Trouvé : 54,68 8,72 12,71

**Exemple 19 : Chlorhydrate de 1-(prop-2-ynyl 1,2,5,6-tétrahydropyridin-3-carboxaldéhyde 0-méthyloxime.**

On opère comme à l'exemple 17 en utilisant du bromure de propynyle et obtient le chlorhydrate attendu. F = 229¤C (décomposition).

Analyse : $C_{10}H_{14}N_2O$, HCl : 214,696
Calculé : C% 55,94 H% 7,04 N% 13,05
Trouvé : 56,02 7,07 12,88

**Exemple 20 : Chlorhydrate de 1-cyclopentyl 1,2,5,6-tétrahydropyridin-3-carboxaldéhyde 0-méthyloxime.**

On opère comme à l'exemple 18 en utilisant du bromure de cyclopentyle en chauffant à 60¤C pendant 8 heures et obtient le produit attendu. F = 213¤C.

Analyse : $C_{12}H_{20}N_2O$, HCl : 244,766
Calculé : C% 58,89 H% 8,65 N% 11,45
Trouvé : 58,62 8,49 11,38

**Exemple 21 : Chlorhydrate de 1,2,5,6-tétrahydropyridin-3-carboxaldéhyde 0-propargyloxime.**

On opère comme à l'exemple 12 à partir de 1,47 g de chlorhydrate de 1,2,5,6-tétrahydropyridin-3-carboxaldéhyde (Chem. Ber. 40, 4685 (1907)) et de 1,07 g de chlorhydrate de 0-propargylhydroxylamine (US patent 3,398,180). On obtiennt 1,2 g de produit attendu. F = 202¤C.

Analyse : $C_9H_{12}N_2O$, HCl : 200,676
Calculé : C% 53,87 H% 6,5 N% 14,12
Trouvé : 53,64 6,53 13,96

**Exemple 22 : Chlorhydrate de 1-méthyl 1,2,5,6-tétrahydropyridin-3-carboxaldéhyde 0-allyloxime.**

A une solution de 0,5 g de sodium dans 20 cm3 d'éthanol anhydre, on ajoute 2,7 g de N-méthyle 1,2,5,6-tétrahydropyridin-3-aldoxime (J. Pharm. Sciences 56 (9), 1190, 1967) puis lentement 1,67 cm3 de bromure d'allyle. On chauffe aureflux pendant 3 heures, verse dans 60 cm3 d'eau, extrait au chlorure de méthylène et lave la phase organique à l'eau salée, sèche et concentre à sec. On chromatographie le résidu sur silice en éluant avec un mélange acétate d'éthyle-méthanol (95-5). On obtient 1 g d'une huile qui distille à 125-130¤C sous 5 mm Hg. On dissout cette huile dans l'éther anhydre, salifie avec de l'acide chlorhydrique gazeux. On recristallise le chlorhydrate obtenu avec un mélange méthanol-éther éthylique. F = 168-169¤C (décomposition).

Analyse : $C_{10}H_{16}N_2O$, HCl : 216,712
Calculé : C% 55,42 H% 7,91 N% 12,93
Trouvé : 55,14 7,82 12,76

**Exemple 23 : Chlorhydrate de 1-méthyl 1,2,5,6-tétrahydropyridin-3-carboxaldéhyde 0-butèn-2-yloxime.**

On opère comme à l'exemple 22 à partir de 3,5 cm3 de bromure de crotyle et 3,8 g de 1-méthyl-1,2,5,6-tétrahydropyridin-3-aldoxime en chauffant 4 heures au reflux. On obtient 1,78 g d'une huile qui distille à 160-165¤C sous 3 mm Hg. On prépare le chlorhydrate qui fond à 164-165¤C (décomposition).
Analyse : $C_{11}H_{18}N_2O$, HCl : 230,739
Calculé : C% 57,26 H% 8,30 N% 12,14
Trouvé : 54,04 8,28 11,99

**ETUDE PHARMACOLOGIOUE**

**Toxicité aigüe.**

L'essai est réalisé sur des souris mâles ($CD_1$ Charles Rivers) de 22 à 24 g, à jeun depuis 16 heures. On administre les produits par voie orale à la dose de 1000, 500, 250, 125, 62, 31 et 16 mg/kg. On note la mortalité pendant les 7 jours suivant le traitement.

| Exemple | $DL_{50}$ mg/kg |
|---|---|
| 1 | 450 |
| 2 | 250 |
| 3 | 75 |
| 4 | 350 |
| 5 | 500 |
| 6 | 750 |
| 7 | 60 |
| 8 | 100 |
| 9 | 30 |
| 10 | 175 |
| 11 | 175 |
| 12 | 60 |
| Arécoline | 600 |

**Test de l'iléon isolé de cobaye.**

On prélève des fragments d'iléon de cobayes tués par décapitation. L'iléon isolé est placé dans 10 cm3 de solution de Tyrode à 37¤C et aéré par un mélange d'oxygène (95%) et de gaz carbonique (5%). Les contractions dues aux produits sont enregistrées à l'aide d'un capteur relié à un polygraphe. Les produits à tester sont ajoutés, aux concentrations comprises entre $1.10^{-3}$M et $1.10^{-8}$ M/l.

Les produits présentant un effet contracturant sont testés vis à vis de l'atropine et de l'hexaméthonium pour établir si l'activité est de type "muscarinique"ou"nicotinique".

L'activité antagoniste éventuelle des produits est testée vis à vis de l'acétylcholine.

L'activité agoniste est exprimée en $pD_2$ (logarithme négatif de la dose qui produit 50% de l'effet maximum).

L'activité antagoniste est exprimée en $DE_{50}$ (dose réduisant de 50% la réponse maximale induite par l'acétylcholine).

| Exemple | $pD_2$ | $DE_{50}$ mg/kg |
|---|---|---|
| 1 | 6,28 | - |
| 2 | <3 | $1,8 \times 10^{-4}$ |
| 3 | 6,58 | - |
| 4 | <3 | $1,1 \times 10^{-5}$ |
| 5 | 4,29 | - |
| 6 | 3,83 | - |
| 7 | <3 | $2,0 \times 10^{-4}$ |
| 8 | 4,60 | - |
| 9 | <3 | $2,3 \times 10^{-4}$ |
| 10 | 4,80 | - |
| 11 | <3 | $1,6 \times 10^{-4}$ |
| 12 | 6,32 | - |
| Arécoline | 6,48 | - |

## Activité diarrhéique.

Le test est réalisé sur des souris mâles ($CD_1$ Charles Rivers) pesant 25 à 30 g, à jeun depuis 6 heures. Le produit dissous à 5% dans du méthocel est administré par voie orale, au moyen d'une sonde oesophagienne.

Des animaux témoins ne reçoivent que l'excipient.

Après traitement, les animaux sont mis séparément dans des cages dont le fond est recouvert de papier buvard et sont mis en observation pendant 30, 60, 120 et 180 minutes.

Les feuilles de papier absorbant sont changées après chaque observation.

La consistance des fèces est évaluée selon la méthode de Randall et Baruth (Arch. Int. Pharmacodyn. 220, 94, 1976) en suivant l'échelle des valeurs suivantes.

0 : consistance ferme,
1 : fèces légèrement molles avec ou sans auréole humide,
2 : fèces légèrement molles avec présence d'un cercle humide bien défini,
3 : fèces molles avec présence d'un grand cercle humide,
4 : fèces sans consistance avec présence d'un très grand cercle humide.

Pour chaque produit, on a noté la dose qui provoque une diarrhée chez 50% des animaux selon la méthode de Miller et Tainter (Proc. Soc. Exp. Biol. Med., 57, 261, 1944).

| Exemple | $DE_{50}$ mg/kg |
|---|---|
| 1 | 0,6 |
| 2 | 50 |
| 3 | 0,15 |
| 4 | > 100 |
| 5 | >100 |
| 6 | >100 |
| 7 | 10 |
| 8 | 1,7 |
| 9 | 3,5 |
| 10 | 1,2 |
| 11 | 5 |
| 12 | 5,5 |
| Arécoline | 35 |

**Activité hypothermique.**

Le test est réalisé sur des souris mâles ($CD_1$ Charles Rivers) pesant 25 à 30 g, à jeun depuis 6 heures.

La température du corps est notée au moyen d'un thermocouple placé dans le rectum à environ 1,5 cm, et relié à un enregistreur de température électrique.

Les produits sont administrés par voie orale ou sous-cutanée et les températures sont notées à l'instant 0 et 30 minutes, 1 heure, 2 heures et 2 heures et demie après traitement.

On évalue le degré d'hypothermie comme — la différence entre les animaux traités et les témoins et on détermine la dose nécessaire pour réduire de 1¤C la température du corps.

| Exemple | Dose efficace (-1¤C) en mg/kg | |
|---|---|---|
| | V.O | V.SC |
| 1 | 0,46 | 0,14 |
| 2 | 13 | 11 |
| 3 | 0,11 | 0,12 |
| 4 | 40 | - |
| 5 | 39 | - |
| 6 | 135 | - |
| 7 | 0,97 | 1,16 |
| 8 | 0,34 | 0,83 |
| 9 | 0,79 | 0,85 |
| 10 | 0,34 | 0,86 |
| 11 | 3 | 4 |
| 12 | 2 | 0,63 |
| Arécoline | 194 | 3 |

On détermine ta durée d'action des produits en utilisant des doses capables de réduire la température de 1¤ à 1,5¤C.

Variations de la température du corps (°C)

| Exemple | dose mg/kg | administration | temps en minute de traitement 0 | 30 | 60 | 120 | 180 |
|---|---|---|---|---|---|---|---|
| 3 | 0,15 | os | + 0,1 | - 1,5** | - 0,8** | - 0,2 | ± 0 |
| | 0,15 | sc | ± 0 | - 1,2** | - 0,8** | - 0,2 | - 0,1 |
| 7 | 1,25 | os | ± 0 | - 1,1** | - 1,1** | - 0,6** | - 0,2 |
| | 1,25 | sc | ± 0 | - 0,5** | - 1,0** | - 0,6** | - 0,1 |
| 8 | 0,5 | os | ± 0 | - 1,5** | - 1,3** | - 0,4** | ± 0 |
| | 1 | sc | ± 0 | - 0,7** | - 1,2** | - 0,2 | - 0,1 |
| 10 | 0,5 | os | - 0,1 | - 1,5** | - 0,7** | - 0,1 | + 0,2 |
| | 0,75 | sc | ± 0 | - 0,8** | - 1,0** | - 0,1 | + 0,1 |
| Arécoline | 200 | os | + 0,1 | - 1,1** | - 1,0** | - 0,2 | - 0,1 |
| | 3,5 | sc | - 0,1 | - 1,5** | - 0,1 | + 0,2 | + 0,2 |

** Valeurs différentes des témoins d'une manière significative ( p < 0,01)

## Revendications

**Revendications pour les Etats contractants suivantes : BE, CH, DE, FR, GB, IT, LI, LU, NL, SE**

**1.** Les composés de formule (I) :

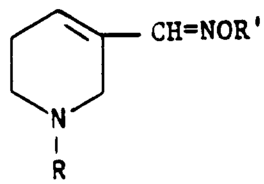

(I)

dans laquelle R représente un atome d'hydrogène, un radical alcoyle linéaire, ramifié ou cyclique, saturé ou insaturé, renfermant jusqu'à 8 atomes de carbone, éventuellement substitué par un radical méthoxycarbonyle, éthoxycarbonyle, propoxycarbonyle linéaire ou ramifié, ou butoxycarbonyle linéaire ou ramifié, ou R représente un radical aralcoyle renfermant jusqu'à 10 atomes de carbone et R' représente un radical alcoyle, linéaire ou ramifié, saturé ou insaturé, renfermant jusqu'à 8 atomes de carbone, un radical -COalc$_1$ ou un radical $-(CH_2)_2N(alc_2)_2$, alc$_1$ et alc$_2$ représentant un radical alcoyle renfermant jusqu'à 8 atomes de carbone, ainsi que leurs sels d'addition avec les acides.

**2.** Les composés de formule (I) tels que définis à la revendication 1, dans lesquels R représente un atome d'hydrogène, ainsi que leurs sels d'addition avec les acides.

**3.** Les composés de formule (I) tels que définis à la revendication 1, dans lesquels R représente un radical alcoyle saturé ou insaturé renfermant ce 1 à 4 atomes de carbone, ainsi que leurs sels d'addition avec les acides

**4.** Les composés de formule (I) tels que définis à la revendication 3, dans lesquels R représente un radical méthyle, éthyle, propyle ou allyle, ainsi que leurs sels d'addition avec les acides.

**5.** Les composés de formule (I) tels que définis à la revendication 1 à 4, dans lesquels R' représente un radical méthyle ainsi que leurs sels a'addition avec les acides.

**6.** Les composés de formule telle que définie à la revendication 1, dont les noms suivent :
- le 1-méthyl 1,2,5,6-tétrahydropyridin-3-carboxaldéhyde 0-méthyl oxime ;
- le 1-éthyl 1,2,5,6-tétrahydropyridin-3-carboxaldéhyde 0-méthyl oxime ;
- le 1,2,5,6-tétrahydropyridin-3-carboxaldéhyde 0-méthyl oxime ;
- le 1-propyl 1,2,5,6-tétrahydropyridin-3-carboxaldéhyde 0-méthyl oxime ;
- le 1-allyl 1,2,5,6-tétrahydropyridin-3-carboxaldéhyde 0-méthyl oxime ;

ainsi que leurs sels et notamment leurs chlorhydrates.

**7.** A titre de médicament, les composés de formule (I) tels que définis à l'une quelconque des revendications 1 à 5, ainsi que leurs sels d'addition avec les acides pharmaceutiquement acceptables.

**8.** A titre de médicament, les composés définis à la revendication 6, ainsi que leurs sels d'addition avec les acides pharmaceutiquement acceptables.

**9.** Les compositions pharmaceutiques renfermant comme principe actif au moins l'un des médicaments définis à la revendication 7 ou 8.

**10.** Procédé de préparation des composés de formule (I), telle que définie à la revendication 1, caractérisé en ce que l'on soumet un composé de formule (II) :

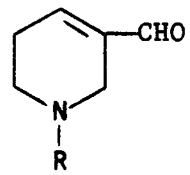

(II)

ou l'un de ses sels,
dans laquelle R conserve la même signification que dans la revendication 1, à l'action d'un composé de formule (III) :

$NH_2OR'_1$ (III)

ou l'un de ses sels,
dans laquelle $R'_1$ représente un atome d'hydrogène ou un radical alcoyle linéaire ou ramifié, saturé ou insaturé, renfermant jusqu'à 8 atomes de carbone, pour obtenir le composé de formule ($I_A$) correspondant :

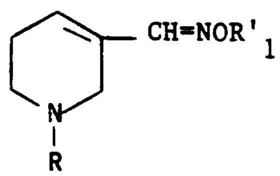

($I_A$)

que l'on salifie le cas échéant, ou que l'on soumet, dans le cas où $R'_1$ représente un atome d'hydrogène, à l'action d'un composé de formule (IV) :

$R'_2Hal$ (IV)

$R'_2$ représentant un radical $COalc_1$ ou un radical $-(CH_2)_2N-(alc_2)_2$, $alc_1$ et $alc_2$ étant définis comme précédemment et Hal représentant un atome d'halogène, pour obtenir le composé de formule ($I_B$) correspondant :

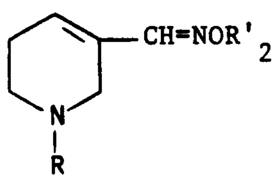

($I_B$)

que l'on salifie le cas échéant, composé de formule ($I_A$) dans lequel $R'_1$ ne représente pas un atome d'hydrogène ou composé de formule ($I_B$) que l'on soumet, le cas échéant, dans le cas où R représente un atome d'hydrogène, à l'action d'un composé de formule (VI) :

$R_1Hal$ (VI)

dans lequel Hal représente un atome d'halogène et $R_1$ représente un radical alcoyle, linéaire, ramifié ou cyclique, saturé ou insaturé, renfermant jusqu'à 8 atomes de carbone, éventuellement substitué par un radical carboxy estérifié ou $R_1$ représente un radical aralcoyle renfermant jusqu'à 10 atomes de carbone , pour obtenir un composé de formule ($I_C$) :

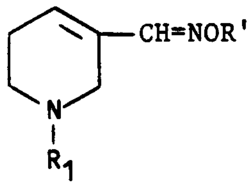

$(I_C)$

dans lequel $R_1$ et $R'$ ont la signification indiquée précédemment, que le cas échéant, l'on salifie, ou que l'on soumet, dans le cas où $R_1$ représente un radical alcoyle substitué par un radical carboxy estérifié, à l'action d'un agent d'hydrolyse pour obtenir le composé correspondant comportant un radical carboxy libre, composé que, le cas échéant, l'on salifie.

**11.** Variante du procédé selon la revendication 10, caractérisé en ce que l'on soumet un composé de formule (V) :

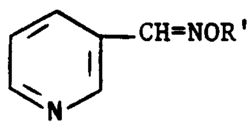

(V)

dans laquelle $R'$ conserve la même signification que dans la revendication 1, à l'action d'un composé de formule (VI) tel que défini à la revendication 10, pour obtenir le composé de formule (VII) :

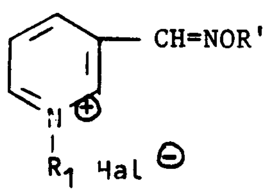

(VII)

que l'on soumet à l'action d'un agent d'hydrogénation, pour obtenir le composé de formule $(I_C)$ correspondant :

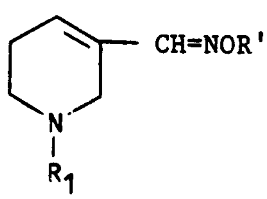

$(I_C)$

dans lequel $R_1$ et $R'$ ont la signification indiquée précédemment que, le cas échéant, l'on salifie ou l'on soumet, dans le cas où $R_1$ représente un radical alcoyle substitué par un radical carboxy estérifié, à l'action d'un agent d'hydrolyse pour obtenir le composé correspondant comportant un radical carboxy libre, composé que le cas échéant, l'on salifie.

**Revendications pour l'Etat contractant suivant : AT**

1. Procédé pour préparer les composés de formule (I) :

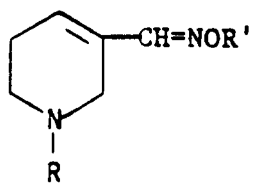

(I)

dans laquelle R représente un atome d'hydrogène, un radical alcoyle linéaire, ramifié ou cyclique, saturé ou insaturé, renfermant jusqu'à 8 atomes de carbone, éventuellement substitué par un radical méthoxycarbonyle, éthoxycarbonyle propoxycarbonyle linéaire ou ramifié, ou butoxycarbonyle linéaire ou ramifié, ou R représente un radical aralcoyle renfermant jusqu'à 10 atomes de carbone et R' représente un radical alcoyle, linéaire ou ramifié, saturé ou insaturé, renfermant jusqu'à 8 atomes de carbone, un radical -COalc$_1$ ou un radical $-(CH_2)_2N(alc_2)_2$, alc$_1$ et alc$_2$ représentant un radical alcoyle renfermant jusqu'à 8 atomes de carbone, ainsi que leurs sels a'addition avec les acides, caractérisé en ce que l'on soumet un composé de formule (II) :

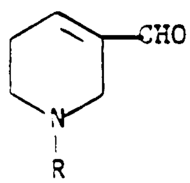

(II)

ou d'un de ses sels,
dans laquelle R conserve la même signification que précédemment, à l'action d'un composé ce formule (III) :

$NH_2OR'_1$ (III)

ou d'un de ses sels,
dans laquelle R'$_1$ représence un atome d'hydrogène ou un radical alcoyle linéaire ou ramifié, saturé ou insaturé, renfermant jusau'à 3 atomes de carbone, pour obtenir le composé de formule ($I_A$) correspondant :

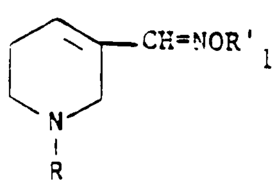

($I_A$)

que l'on salifie le cas échéant, ou que l'on soumet, dans le cas où R'$_1$ représente un atome d'hydrogène, à l'action d'un composé de formule (IV) :

$R'_2Hal$ (IV)

$R'_2$ représentant un radical COalc$_1$ ou un radical $-(CH_2)_2N-(alc_2)_2$, alc$_1$ et alc$_2$ étant définis comme précédemment et Hal représentant un atome d'halogène, pour obtenir le composé de formule ($I_B$) correspondant :

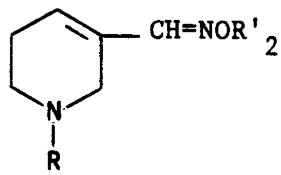

$(I_B)$

que l'on salifie le cas échéant, composé de formule $(I_A)$ dans lequel $R'_1$ ne représente pas un atome d'hydrogène ou composé de formule $(I_B)$ que l'on soumet, le cas échéant, dans le cas où R représente un atome d'hydrogène, à l'action d'un composé de formule (VI) :

$R_1Hal$ (VI)

dans lequel Hal représente un atome d'halogène et $R_1$ représente un radical alcoyle, linéaire, ramifié ou cyclique, saturé ou insaturé, renfermant jusqu'à 8 atomes de carbone, éventuellement substitué par un radical carboxy estérifié ou $R_1$ représente un radical aralcoyle renfermant jusqu'à 10 atomes de carbone, pour obtenir un composé de formule $(I_C)$ :

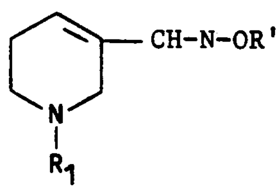

$(I_C)$

dans lequel $R_1$ et $R'$ ont la signification indiquée précédemment, que le cas échéant, l'on salifie, ou que l'on soumet, dans le cas où $R_1$ représente un radical alcoyle substitué par un radical carboxy estérifié, à l'action d'un agent d'hydrolyse pour obtenir le composé correspondant comportant un radical carboxy libre, composé que, le cas échéant, l'on salifie.

**2.** Procédé selon la revendication 1, pour préparer les produits répondant à la formule $(I_1)$ :

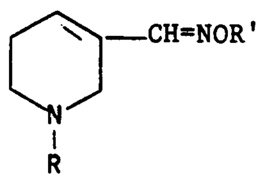

$(I_1)$

dans laquelle R représente un atome d'hydrogène ou un radical alcoyle, linéaire ou ramifié, saturé ou insaturé renfermant jusqu'à 8 atomes de carbone et $R'$ a la signification déjà indiquée, ainsi que leurs sels d'addition avec les acides, caractérisé en ce que l'on soumet un composé de formule (II) :

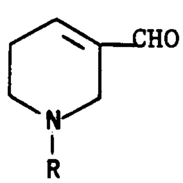

(II)

ou l'un de ses sels,
dans laquelle R conserve la même signification que précédemment, à l'action d'un composé de formule (III) :

$NH_2OR'_1$ (III)

ou l'un de ses sels,
dans laquelle $R'_1$ représente un atome d'hydrogène ou un radical alcoyle linéaire ou ramifié, saturé ou insaturé, renfermant jusqu'à 8 atomes de carbone, pour obtenir le composé de formule ($I_A$) correspondant :

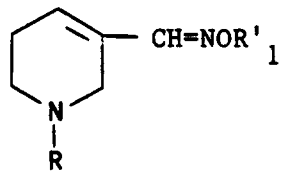

($I_A$)

que l'on salifie le cas échéant, ou que l'on soumet, dans le cas où $R'_1$ représente un atome d'hydrogène, à l'action d'un composé de formule (IV) :

$R'_2Hal$ (IV)

$R'_2$ représentant un radical $COalc_1$ ou un radical $-(CH_2)_2N-(alc_2)_2$, $alc_1$ et $alc_2$ étant définis comme précédemment et Hal représentant un atome d'halogène, pour obtenir le composé de formule ($I_B$) correspondant :

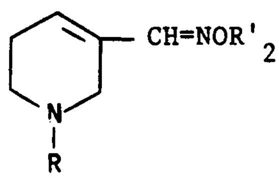

($I_B$)

que l'on salifie le cas échéant.

**3.** Variante du procédé selon la revendication 1, caractérisé en ce que l'on soumet un composé de formule (V) :

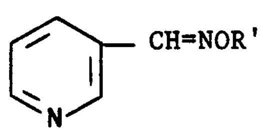

(V)

dans laquelle R' conserve la même signification que dans la revendication 1, à l'action d'un composé de formule (VI) tel que défini à la revendication 10, pour obtenir le composé de formule (VII) :

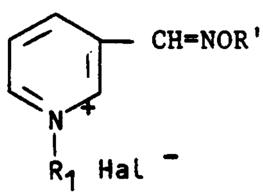

(VII)

que l'on soumet à l'action d'un agent d'hydrogénation, pour obtenir le composé de formule ($I_C$) correspondant :

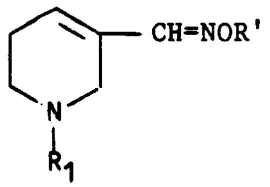

($I_C$)

dans lequel $R_1$ et $R'$ ont la signification indiquée précédemment que, le cas échéant, l'on salifie ou l'on soumet, dans le cas où $R_1$ représente un radical alcoyle substitué par un radical carboxy estérifié, à l'action d'un agent d'hydrolyse pour obtenir le composé correspondant comportant un radical carboxy libre, composé que le cas échéant, l'on salifie.

**4.** Variante du procédé selon la revendication 2, caractérisé en ce que l'on soumet un composé de formule (V) :

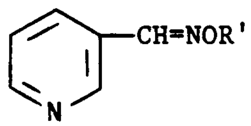

(V)

dans laquelle $R'$ conserve la même signification que dans la revendication 1, à l'action d'un composé de formule (VI) tel que défini à la revendication 10, pour obtenir le composé de formule (VII) :

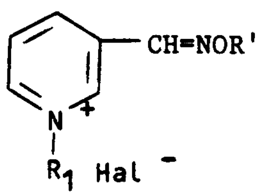

(VII)

que l'on soumet à l'action d'un agent d'hydrogénation, pour obtenir le composé de formule ($I_C$) correspondant :

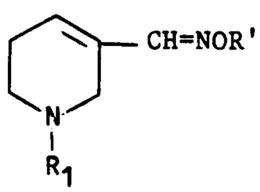

($I_C$)

que l'on salifie, le cas échéant.

**5.** Procédé selon la revendication 2, caractérisé en ce que l'on utilise au départ un composé de formule (II) dans laquelle R représente un atome d'hydrogène.

**6.** Procédé selon la revendication 2, caractérisé en ce que l'on utilise au départ un composé de formule (II) dans laquelle R représente un radical alcoyle saturé ou insaturé renfermant de 1 à 4 atomes de carbone.

**7.** Procédé selon la revendication 2, caractérisé en ce que l'on utilise au départ un composé de formule (II) dans laquelle R représente un radical méthyle, éthyle, propyle ou allyle.

**8.** Procédé selon la revendication 2, caractérisé en ce que l'on utilise au départ un composé de formule (III) dans laquelle $R'_1$ représente un radical méthyle.

9. Procédé selon la revendication 2, caractérisé en ce que l'on prépare l'un quelconque des produits de formule ($I_1$) dont les noms suivent :
   - le 1-méthyl 1,2,5,6-tétrahydropyridin-3-carboxaldéhyde 0-méthyl oxime ;
   - le 1-éthyl 1,2,5,6-tétrahydropyridin-3-carboxaldéhyde 0-méthyl oxime ;
   - le 1,2,5,6-tétrahydropyridin-3-carboxaldéhyde 0-méthyl oxime ;
   - le 1-propyl 1,2,5,6-tétrahydropyridin-3-carboxaldéhyde 0-méthyl oxime ;
   - le 1-allyl 1,2,5,6-tétrahydropyridin-3-carboxaldéhyde 0-méthyl oxime ;

   ainsi que leurs sels et notamment leurs chlorhydrates.

**Revendications pour l'Etat contractant suivant : ES**

1. Procédé pour préparer les composés de formule (I) :

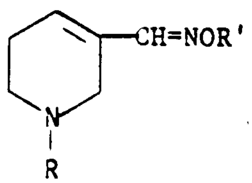

(I)

dans laquelle R représente un atome d'hydrogène, un radical alcoyle linéaire, ramifié ou cyclique, saturé ou insaturé, renfermant jusqu'à 8 atomes de carbone, éventuellement substitué par un radical méthoxycarbonyle, éthoxycarbonyle, propoxycarbonyle linéaire ou ramifié, ou butoxycarbonyle linéaire ou ramifié, ou R représente un radical aralcoyle renfermant jusqu'à 10 atomes de carbone et R' représente un radical alcoyle, linéaire ou ramifié, saturé ou insaturé, renfermant jusqu'à 8 atomes de carbone, un radical $-COalc_1$ ou un radical $-(CH_2)_2N(alc_2)_2$, $alc_1$ et $alc_2$ représentant un radical alcoyle renfermant jusqu'à 8 atomes de carbone, ainsi que leurs sels d'addition avec les acides, caractérisé en ce que l'on soumet un composé de formule (II) :

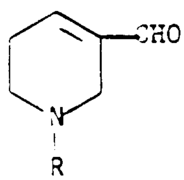

(II)

ou l'un de ses sels,
dans laquelle R conserve la même signification que précédemment, à l'action d'un composé de formule (III) :

$NH_2OR'_1$ (III)

ou d'un de ses sels,
dans laquelle $R'_1$ représente un atome d'hydrogène ou un radical alcoyle linéaire ou ramifié, saturé ou insaturé, renfermant jusqu'à 3 atomes de carbone, pour obtenir le composé de formule ($I_A$) correspondant :

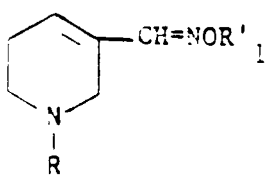

($I_A$)

que l'on salifie le cas échéant, ou que l'on soumet, dans le cas où $R'_1$ représente un atome d'hydrogène, à l'action d'un composé de formule (IV) :

$R'_2Hal$ (IV)

$R'_2$ représentant un radical $COalc_1$ ou un radical $-(CH_2)_2N-(alc_2)_2$, $alc_1$ et $alc_2$ étant définis comme précédemment et Hal représentant un atome d'halogène, pour obtenir le composé de formule ($I_B$) correspondant :

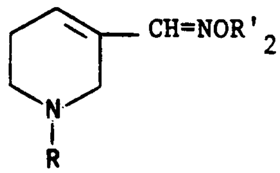

($I_B$)

que l'on salifie le cas échéant, composé de formule ($I_A$) dans lequel $R'_1$ ne représente pas un atome d'hydrogène ou composé de formule ($I_B$) que l'on soumet, le cas échéant, dans le cas où R représente un atome d'hydrogène, à l'action d'un composé de formule (VI) :

$R_1$ Hal (VI)

dans lequel Hal représente un atome d'halogène et $R_1$ représente un radical alcoyle, linéaire, ramifié ou cyclique, saturé ou insaturé, renfermant jusqu'à 8 atomes de carbone, éventuellement substitué par un radical carboxy estérifié ou $R_1$ représente un radical aralcoyle renfermant jusqu'à 10 atomes de carbone, pour obtenir un composé de formule ($I_C$) :

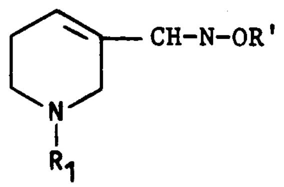

($I_C$)

dans lequel $R_1$ et $R'$ ont la signification indiquée précédemment, que le cas échéant, l'on salifie, ou que l'on soumet, dans le cas où $R_1$ représente un radical alcoyle substitué par un radical carboxy estérifié, à l'action d'un agent d'hydrolyse pour obtenir le composé correspondant comportant un radical carboxy libre, composé que, le cas échéant, l'on salifie.

**2.** Procédé selon la revendication 1, pour préparer les produits répondant à la formule ($I_1$) :

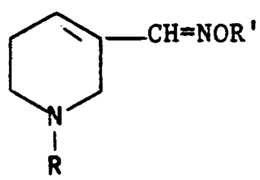

($I_1$)

dans laquelle R représente un atome d'hydrogène ou un radical alcoyle, linéaire ou ramifié, saturé ou insaturé renfermant jusqu'à 8 atomes de carbone et $R'$ a la signification déjà indiquée, ainsi que leurs sels d'addition avec les acides, caractérisé en ce que l'on soumet un composé de formule (II) :

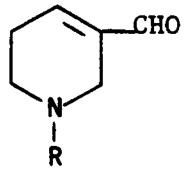

(II)

ou l'un de ses sels,
dans laquelle R conserve la même signification que précédemment, à l'action d'un composé de

formule (III) :

$NH_2OR'_1$ (III)

ou l'un de ses sels,
dans laquelle $R'_1$ représente un atome d'hydrogène ou un radical alcoyle linéaire ou ramifié, saturé ou insaturé, renfermant jusqu'à 8 atomes de carbone, pour obtenir le composé de formule ($I_A$) correspondant :

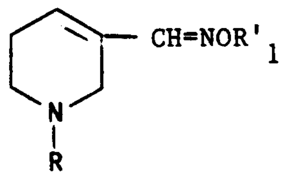

($I_A$)

que l'on salifie le cas échéant, ou que l'on soumet, dans le cas où $R'_1$ représente un atome d'hydrogène, à l'action d'un composé de formule (IV) :

$R'_2Hal$ (IV)

$R'_2$ représentant un radical $COalc_1$ ou un radical $-(CH_2)_2N-(alc_2)_2$, $alc_1$ et $alc_2$ étant définis comme précédemment et Hal représentant un atome d'halogène, pour obtenir le composé de formule ($I_B$) correspondant :

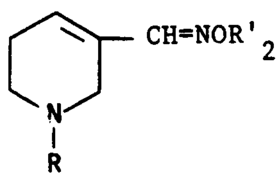

($I_B$)

que l'on salifie le cas échéant.

**3.** Variante du procédé selon la revendication 1, caractérisé en ce que l'on soumet un composé de formule (V) :

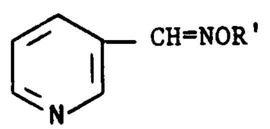

(V)

dans laquelle R′ conserve la même signification que dans la revendication 1, à l'action d'un composé de formule (VI) tel que défini à la revendication 10, pour obtenir le composé de formule (VII) :

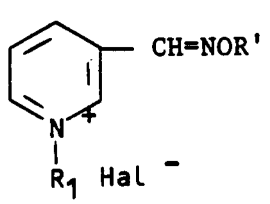

(VII)

que l'on soumet à l'action d'un agent d'hydrogénation, pour obtenir le composé de formule ($I_C$) correspondant :

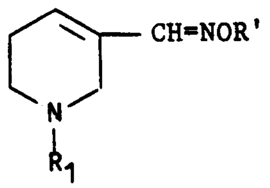

$(I_C)$

dans lequel $R_1$ et $R'$ ont la signification indiquée précédemment que, le cas échéant, l'on salifie ou l'on soumet, dans le cas où $R_1$ représente un radical alcoyle substitué par un radical carboxy estérifié, à l'action d'un agent d'hydrolyse pour obtenir le composé correspondant comportant un radical carboxy libre, composé que le cas échéant, l'on salifie.

**4.** Variante du procédé selon la revendication 2, caractérisé en ce que l'on soumet un composé de formule (V) :

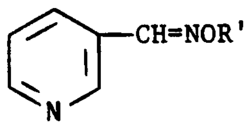

(V)

dans laquelle $R'$ conserve la même signification que dans la revendication 1, à l'action d'un composé de formule (VI) tel que défini à la revendication 10, pour obtenir le composé de formule (VII) :

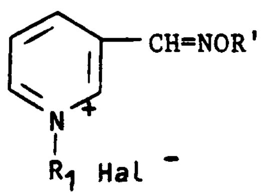

(VII)

que l'on soumet à l'action d'un agent d'hydrogénation, pour obtenir le composé de formule $(I_C)$ correspondant :

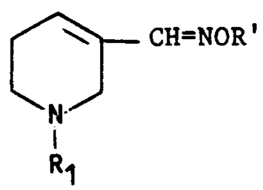

$(I_C)$

que l'on salifie, le cas échéant.

**5.** Procédé selon la revendication 2, caractérisé en ce que l'on utilise au départ un composé de formule (II) dans laquelle R représente un atome d'hydrogène.

**6.** Procédé selon la revendication 2, caractérisé en ce que l'on utilise au départ un composé de formule (II) dans laquelle R représente un radical alcoyle saturé ou insaturé renfermant de 1 à 4 atomes de carbone.

**7.** Procédé selon la revendication 2, caractérisé en ce que l'on utilise au départ un composé de formule (II) dans laquelle R représente un radical méthyle, éthyle, propyle ou allyle.

**8.** Procédé selon la revendication 2, caractérisé en ce que l'on utilise au départ un composé de formule (III) dans laquelle $R'_1$ représente un radical méthyle.

9. Procédé selon la revendication 2, caractérisé en ce que l'on prépare l'un quelconque des produits de formule (I1) dont les noms suivent :
   - le 1-méthyl 1,2,5,6-tétrahydropyridin-3-carboxaldéhyde 0-méthyl oxime ;
   - le 1-éthyl 1,2,5,6-tétrahydropyridin-3-carboxaldéhyde 0-méthyl oxime ;
   - le 1,2,5,6-tétrahydropyridin-3-carboxaldéhyde 0-méthyl oxime ;
   - le 1-propyl 1,2,5,6-tétrahydropyridin-3-carboxaldéhyde 0-méthyl oxime ;
   - le 1-allyl 1,2,5,6-tétrahydropyridin-3-carboxaldéhyde 0-méthyl oxime ;

   ainsi que leurs sels et notamment leurs chlorhydrates.

**Revendications pour l'Etat contractant suivant : GR**

1. Procédé pour préparer les composés de formule (I) :

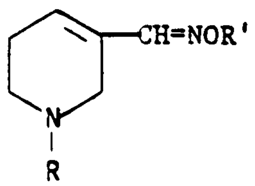

(I)

dans laquelle R représente un atome d'hydrogène, un radical alcoyle linéaire, ramifié ou cyclique, saturé ou insaturé, renfermant jusqu'à 8 atomes de carbone, éventuellement substitué par un radical méthoxycarbonyle, éthoxycarbonyle, propoxycarbonyle linéaire ou ramifié, ou butoxycarbonyle linéaire ou ramifié, ou R représente un radical aralcoyle renfermant jusqu'à 10 atomes de carbone et R' représente un radical alcoyle, linéaire ou ramifié, saturé ou insaturé, renfermant jusqu'à 8 atomes de carbone, un radical -COalc$_1$ ou un radical $-(CH_2)_2N(alc_2)_2$, alc$_1$ et alc$_2$ représentant un radical alcoyle renfermant jusqu'à 8 atomes de carbone, ainsi que leurs sels d'addition avec les acides, caractérisé en ce que l'on soumet un composé de formule (II) :

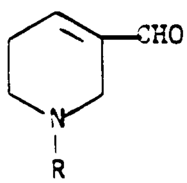

(II)

ou d'un de ses sels,
dans laquelle R conserve la même signification que précédemment, à l'action d'un composé de formule (III) :

$NH_2OR'_1$ (III)

ou d'un de ses sels,
dans laquelle $R'_1$ représente un atome d'hydrogène ou un radical alcoyle linéaire ou ramifié, saturé ou insaturé, renfermant jusqu'à 3 atomes de carbone, pour obtenir le composé de formule ($I_A$) correspondant :

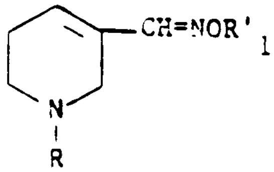

($I_A$)

que l'on salifie le cas échéant, ou que l'on soumet, dans le cas où $R'_1$ représente un atome d'hydrogène, à l'action d'un composé de formule (IV) :

$R'_2Hal$ (IV)

$R'_2$ représentant un radical $COalc_1$ ou un radical $-(CH_2)_2N-(alc_2)_2$, $alc_1$ et $alc_2$ étant définis comme précédemment et Hal représentant un atome d'halogène, pour obtenir le composé de formule ($I_B$) correspondant :

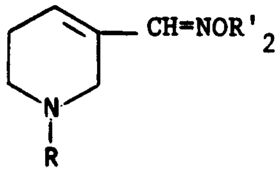

($I_B$)

que l'on salifie le cas échéant, composé de formule ($I_A$) dans lequel $R'_1$ ne représente pas un atome d'hydrogène ou composé de formule ($I_B$) que l'on soumet, le cas échéant, dans le cas où R représente un atome d'hydrogène, à l'action d'un composé de formule (VI) :

$R_1Hal$ (VI)

dans lequel Hal représente un atome d'halogène et $R_1$ représente un radical alcoyle, linéaire, ramifié ou cyclique, saturé ou insaturé, renfermant jusqu'à 8 atomes de carbone, éventuellement substitué par un radical carboxy estérifié ou $R_1$ représente un radical aralcoyle renfermant jusqu'à 10 atomes de carbone, pour obtenir un composé de formule ($I_C$) :

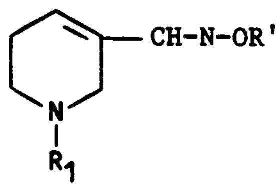

($I_C$)

dans lequel $R_1$ et $R'$ ont la signification indiquée précédemment, que le cas échéant, l'on salifie, ou que l'on soumet, dans le cas où $R_1$ représente un radical alcoyle substitué par un radical carboxy estérifié, à l'action d'un agent d'hydrolyse pour obtenir le composé correspondant comportant un radical carboxy libre, composé que, le cas échéant, l'on salifie.

**2.** Procédé selon la revendication 1, pour préparer les produits répondant à la formule ($I_1$) :

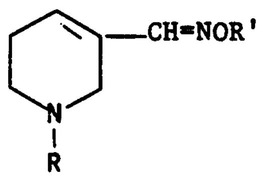

($I_1$)

dans laquelle R représente un atome d'hydrogène ou un radical alcoyle, linéaire ou ramifié, saturé ou insaturé renfermant jusqu'à 8 atomes de carbone et $R'$ a la signification déjà indiquée, ainsi que leurs sels d'addition avec les acides, caractérisé en ce que l'on soumet un composé de formule (II) :

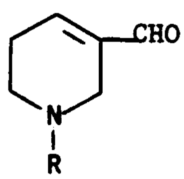

(II)

ou l'un de ses sels,
dans laquelle R conserve la même signification que précédemment, à l'action d'un composé de

formule (III) :

$NH_2OR'_1$ (III)

ou l'un de ses sels,
dans laquelle $R'_1$ représente un atome d'hydrogène ou un radical alcoyle linéaire ou ramifié, saturé ou insaturé, renfermant jusqu'à 8 atomes de carbone, pour obtenir le composé de formule ($I_A$) correspondant :

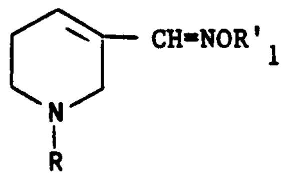

($I_A$)

que l'on salifie le cas échéant, ou que l'on soumet, dans le cas où $R'_1$ représente un atome d'hydrogène, à l'action d'un composé de formule (IV) :

$R'_2Hal$ (IV)

$R'_2$ représentant un radical $COalc_1$ ou un radical $-(CH_2)_2N-(alc_2)_2$, $alc_1$ et $alc_2$ étant définis comme précédemment et Hal représentant un atome d'halogène, pour obtenir le composé de formule ($I_B$) correspondant :

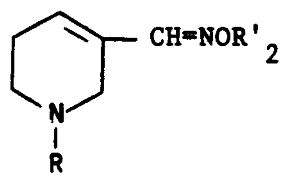

($I_B$)

que l'on salifie le cas échéant.

**3.** Variante du procédé selon la revendication 1, caractérisé en ce que l'on soumet un composé de formule (V) :

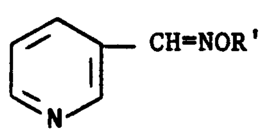

(V)

dans laquelle R' conserve la même signification que dans la revendication 1, à l'action d'un composé de formule (VI) tel que défini à la revendication 10, pour obtenir le composé de formule (VII) :

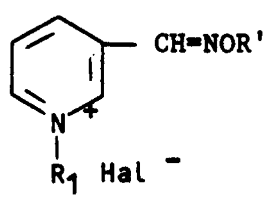

(VII)

que l'on soumet à l'action d'un agent d'hydrogénation, pour obtenir le composé de formule ($I_C$) correspondant :

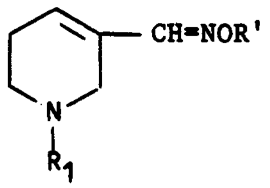

$(I_C)$

dans lequel $R_1$ et $R'$ ont la signification indiquée précédemment que, le cas échéant, l'on salifie ou l'on soumet, dans le cas où $R_1$ représente un radical alcoyle substitué par un radical carboxy estérifié, à l'action d'un agent d'hydrolyse pour obtenir le composé correspondant comportant un radical carboxy libre, composé que le cas échéant, l'on salifie.

**4.** Variante du procédé selon la revendication 2, caractérisé en ce que l'on soumet un composé de formule (V) :

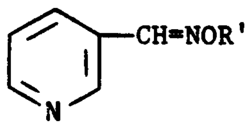

(V)

dans laquelle $R'$ conserve la même signification que dans la revendication 1, à l'action d'un composé de formule (VI) tel que défini à la revendication 10, pour obtenir le composé de formule (VII) :

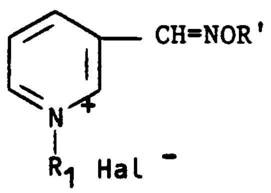

(VII)

que l'on soumet à l'action d'un agent d'hydrogénation, pour obtenir le composé de formule $(I_C)$ correspondant :

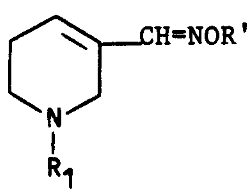

$(I_C)$

que l'on salifie, le cas échéant.

**5.** Procédé selon la revendication 2, caractérisé en ce que l'on utilise au départ un composé de formule (II) dans laquelle R représente un atome d'hydrogène.

**6.** Procédé selon la revendication 2, caractérisé en ce que l'on utilise au départ un composé de formule (II) dans laquelle R représente un radical alcoyle saturé ou insaturé renfermant de 1 à 4 atomes de carbone.

**7.** Procédé selon la revendication 2, caractérisé en ce que l'on utilise au départ un composé de formule (II) dans laquelle R représente un radical méthyle, éthyle, propyle ou allyle.

**8.** Procédé selon la revendication 2, caractérisé en ce que l'on utilise au départ un composé de formule (III) dans laquelle $R'_1$ représente un radical méthyle.

9. Procédé selon la revendication 2, caractérisé en ce que l'on prépare l'un quelconque des produits de formule ($I_1$) dont les noms suivent :
   - le 1-méthyl 1,2,5,6-tétrahydropyridin-3-carboxaldéhyde 0-méthyl oxime ;
   - le 1-éthyl 1,2,5,6-tétrahydropyridin-3-carboxaldéhyde 0-méthyl oxime ;
   - le 1,2,5,6-tétrahydropyridin-3-carboxaldéhyde 0-méthyl oxime ;
   - le 1-propyl 1,2,5,6-tétrahydropyridin-3-carboxaldéhyde 0-méthyl oxime ;
   - le 1-allyl 1,2,5,6-tétrahydropyridin-3-carboxaldéhyde 0-méthyl oxime ;

   ainsi que leurs sels et notamment leurs chlorhydrates.

10. Procédé de préparation de compositions pharmaceutiques, caractérisé en ce que l'on met, à titre de principe actif, l'un au moins des produits de formule (I), telle que définie à la revendication 1, sous une forme destinée à cet usage.

11. Procédé de préparation de compositions pharmaceutiques, caractérisé en ce que l'on met, à titre de principe actif, l'un au moins des produits de formule (I1), telle que définie à la revendication 2, sous une forme destinée à cet usage.

12. Procédé de préparation de compositions pharmaceutiques, caractérisé en ce que l'on met, à titre de principe actif, l'un au moins des produits de formule (I), telle que définie à la revendication 9, sous une forme destinée à cet usage.

**Claims**

**Claims for the following Contracting States : BE, CH, DE, FR, GB, IT, LI, LU, NL, SE**

1. The compounds of formula (I):

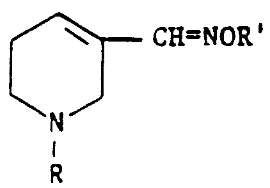

(I)

   in which R represents a hydrogen atom, a saturated or unsaturated, linear, branched or cyclic alkyl radical containing up to 8 carbon atoms, optionally substituted by a methoxycarbonyl, ethoxycarbonyl, linear or branched propoxycarbonyl or linear or branched butoxycarbonyl radical, or R represents an aralkyl radical containing up to 10 carbon atoms and R' represents a saturated or unsaturated, linear or branched alkyl radical containing up to 8 carbon atoms, a $-COalk_1$ radical or a $-(CH_2)_2N(alk_2)_2$ radical, $alk_1$ and $alk_2$ representing an alkyl radical containing up to 8 carbon atoms, as well as their addition salts with acids.

2. The compounds of formula (I) as defined in claim 1, in which R represents a hydrogen atom, as well as their addition salts with acids.

3. The compounds of formula (I) as defined in claim 1, in which R represents a saturated or unsaturated alkyl radical containing 1 to 4 carbon atoms, as well as their addition salts with acids.

4. The compounds of formula (I) as defined in claim 3, in which R represents a methyl, ethyl, propyl or allyl radical, as well as their addition salts with acids.

5. The compounds of formula (I) as defined in claims 1 to 4, in which R' represents a methyl radical, as well as their addition salts with acids.

6. The compounds of formula (I) as defined in claim 1, the names of which follow:
   - 1-methyl-1,2,5,6-tetrahydropyridin-3-carboxaldehyde 0-methyl oxime;
   - 1-ethyl-1,2,5,6-tetrahydropyridin-3-carboxaldehyde 0-methyl oxime;

- 1,2,5,6-tetrahydropyridin-3-carboxaldehyde 0-methyl oxime;
- 1-propyl-1,2,5,6-tetrahydropyridin-3-carboxaldehyde 0-methyl oxime;
- 1-allyl-1,2,5,6-tetrahydropyridin-3-carboxaldehyde 0-methyl oxime;

as well as their salts and notably their hydrochlorides.

**7.** As medicaments, the compounds of formula (I) as defined in any one of claims 1 to 5, as well as their addition salts with pharmaceutically acceptable acids.

**8.** As medicaments, the compounds defined in claim 6, as well as their addition salts with pharmaceutically acceptable acids.

**9.** The pharmaceutical compositions containing as active ingredient at least one of the medicaments defined in claim 7 or 8.

**10.** Preparation process for compounds of formula (I), as defined in claim 1, characterized in that a compound of formula (II):

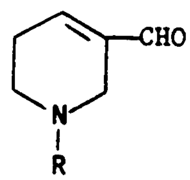

(II)

or one of its salts,
in which R keeps the same meaning as in claim 1, is subjected to the action of a compound of formula (III):

$NH_2$-OR'$_1$ (III)

or one of its salts,
in which R'$_1$ represents a hydrogen atom or a saturated or unsaturated, linear or branched alkyl radical containing up to 8 carbon atoms, in order to obtain the corresponding compound of formula ($I_A$):

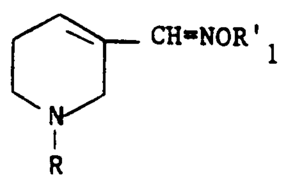

($I_A$)

which if appropriate is salified, or is subjected, in the case where R'$_1$ represents a hydrogen atom, to the action of a compound of formula (IV):

R'$_2$Hal (IV)

R'$_2$ representing a COalk$_1$ radical or a -$(CH_2)_2$N-(alk$_2$)$_2$ radical, alk$_1$ and alk$_2$ being defined as previously and Hal representing a halogen atom, in order to obtain the corresponding compound of formula ($I_B$):

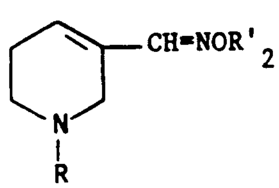

($I_B$)

which if appropriate is salified, in which compound of formula ($I_A$) R'$_1$ does not represent a hydrogen

atom or which compound of formula ($I_B$) is subjected, if appropriate, in the case where R represents a hydrogen atom, to the action of a compound of formula (VI):

$R_1$ Hal (VI)

in which Hal represents a halogen atom and $R_1$ represents a saturated or unsaturated, linear, branched or cyclic alkyl radical containing up to 8 carbon atoms, optionally substituted by an esterified carboxy radical or $R_1$ represents an aralkyl radical containing up to 10 carbon atoms, in order to obtain a compound of formula ($I_C$):

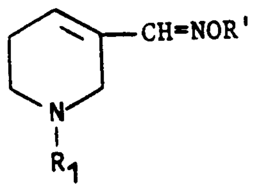

($I_C$)

in which $R_1$ and R' have the meaning indicated previously, which if appropriate is salified, or is subjected, in the case where $R_1$ represents an alkyl radical substituted by an esterified carboxy radical, to the action of a hydrolysis agent in order to obtain the corresponding compound comprising a free carboxy radical, which compound, if appropriate, is salified.

**11.** Variant of the process according to claim 10, characterized in that a compound of formula (V):

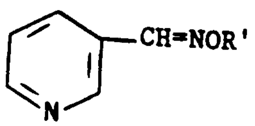

(V)

in which R' keeps the same meaning as in claim 1, is subjected to the action of a compound of formula (VI) as defined in claim 1 , in order to obtain the compound of formula (VII):

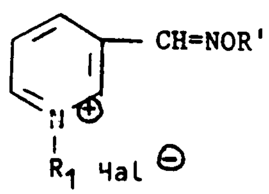

(VII)

which is subjected to the action of a hydrogenation agent, in order to obtain the corresponding compound of formula ($I_C$):

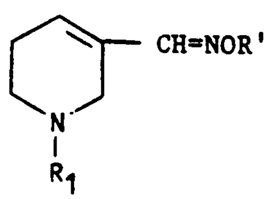

($I_C$)

in which $R_1$ and R' have the meaning indicated previously, which if appropriate is salified, or is subjected, in the case where $R_1$ represents an alkyl radical substituted by an esterified carboxy radical, to the action of a hydrolysis agent in order to obtain the corresponding compound containing a free carboxy radical, which compound, if appropriate, is salified.

**Claims for the following Contracting State : AT**

**1.** Process for preparing the compounds of formula (I):

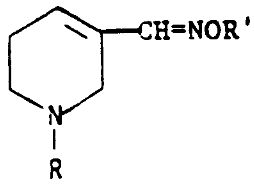

**(I)**

in which R represents a hydrogen atom, a saturated or unsaturated, linear, branched or cyclic alkyl radical containing up to 8 carbon atoms, optionally substituted by a methoxycarbonyl, ethoxycarbonyl, linear or branched propoxycarbonyl or linear or branched butoxycarbonyl radical, or R represents an aralkyl radical containing up to 10 carbon atoms and R' represents a saturated or unsaturated, linear or branched alkyl radical containing up to 8 carbon atoms, a -COalk$_1$ radical or a -$(CH_2)_2N(alk_2)_2$ radical, alk$_1$ and alk$_2$ representing an alkyl radical containing up to 8 carbon atoms, as well as their addition salts with acids,
characterized in that a compound of formula (II):

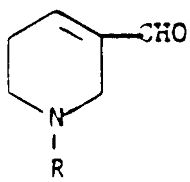

**(II)**

or one of its salts,
in which R keeps the same meaning as previously, is subjected to the action of a compound of formula (III):

$NH_2$-OR'$_1$ (III)

or one of its salts, in which R'$_1$ represents a hydrogen atom or a saturated or unsaturated, linear or branched alkyl radical containing up to 8 carbon atoms, in order to obtain the corresponding compound of formula ($I_A$):

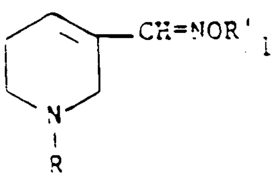

**($I_A$)**

which if appropriate is salified, or is subjected, in the case where R'$_1$ represents a hydrogen atom, to the action of a compound of formula (IV):

R'$_2$Hal (III)

R'$_2$ representing a COalk$_1$ radical or a -$(CH_2)_2$N-(alk$_2$)$_2$ radical, alk$_1$ and alk$_2$ being defined as previously and Hal representing a halogen atom, in order to obtain the corresponding compound of formula ($I_B$):

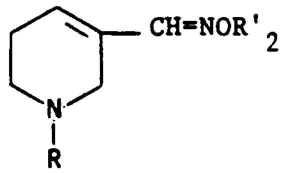

$(I_B)$

which is appropriate is salified, in which compound of formula $(I_A)$ $R'_1$ does not represent a hydrogen atom or which compound of formula $(I_B)$ is subjected, if appropriate, in the case where R represents a hydrogen atom, to the action of a compound of formula (VI):

$R_1$Hal (VI)

in which Hal represents a halogen atom and $R_1$ represents a saturated or unsaturated, linear, branched or cyclic alkyl radical containing up to 8 carbon atoms, optionally substituted by an esterified carboxy radical or $R_1$ represents an aralkyl radical containing up to 10 carbon atoms, in order to obtain a compound of formula $(I_C)$:

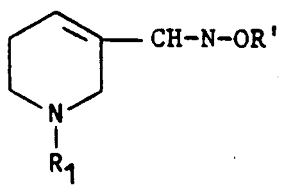

$(I_C)$

in which $R_1$ and R' have the meaning indicated previously, which if appropriate is salified, or is subjected, in the case where $R_1$ represents an alkyl radical substituted by an esterified carboxy radical, to the action of a hydrolysis agent in order to obtain the corresponding compound comprising a free carboxy radical, which compound, if appropriate, is salified.

**2.** Process according to claim 1, for preparing the products corresponding to formula $(I_1)$:

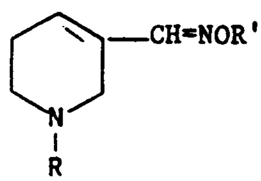

$(I_1)$

in which R represents a hydrogen atom or a saturated or unsaturated, linear or branched alkyl radical containing up to 8 carbon atoms and R' has the meaning already indicated, as well as their addition salts with acids, characterized in that a compound of formula (II):

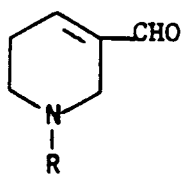

(II)

or one of its salts,
in which R keeps the same meaning as previously, is subjected to the action of a compound of formula (III):

$NH_2OR'_1$ (III)

or one of its salts,

in which $R'_1$ represents a hydrogen atom or a saturated or unsaturated, linear or branched alkyl radical containing up to 8 carbon atoms, in order to obtain the corresponding compound of formula ($I_A$):

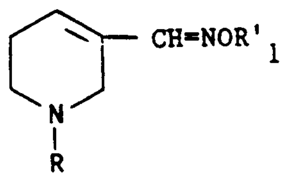

($I_A$)

which if appropriate is salified, or is subjected, in the case where $R'_1$ represents a hydrogen atom, to the action of a compound of formula (IV):

$R'_2Hal$ (IV)

$R'_2$ representing a $COalk_1$ radical or a $-(CH_2)_2N-(alk_2)_2$ radical, $alk_1$ and $alk_2$ being defined as previously and Hal representing a halogen atom, in order to obtain a corresponding compound of formula ($I_B$):

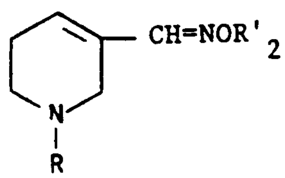

($I_B$)

which if appropriate is salified.

**3.** Variant of the process according to claim 1, characterized in that a compound of formula (V):

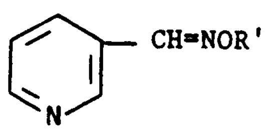

(V)

in which R' keeps the same meaning as in claim 1, is subjected to the action of a compound of formula (VI) as defined in claim 1, in order to obtain the compound of formula (VII):

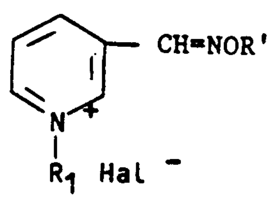

(VII)

which is subjected to the action of a hydrogenation agent, in order to obtain the corresponding compound of formula ($I_C$):

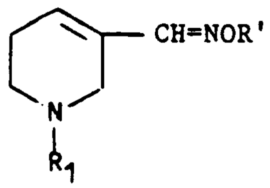

$(I_C)$

in which $R_1$ and R' have the meaning indicated previously, which if appropriate is salified, or is subjected, in the case where $R_1$ represents an alkyl radical substituted by an esterified carboxy radical, to the action of a hydrolysis agent in order to obtain the corresponding compound containing a free carboxy radical, which compound, if appropriate, is salified.

4. Variant of the process according to claim 2, characterized in that a compound of formula (V):

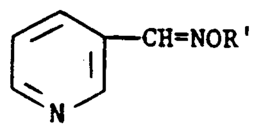

(V)

in which R' keeps the same meaning as in claim 1, is subjected to the action of a compound of formula (VI) as defined in claim 1, in order to obtain the compound of formula (VII):

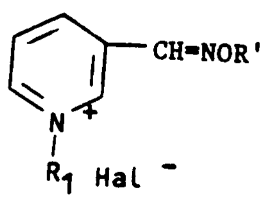

(VII)

which is subjected to the action of a hydrogenation agent, in order to obtain the corresponding compound of formula $(I_C)$:

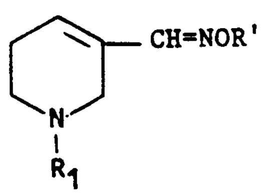

$(I_C)$

which, if appropriate, is salified.

5. Process according to claim 2, characterized in that at the start a compound of formula (II) is used in which R represents a hydrogen atom.

6. Process according to claim 2, characterized in that at the start a compound of formula (II) is used in which R represents a saturated or unsaturated alkyl radical containing 1 to 4 carbon atoms.

7. Process according to claim 2, characterized in that at the start a compound of formula (II) is used in which R represents a methyl, ethyl, propyl or allyl radical.

8. Process according to claim 2, characterized in that at the start a compound of formula (III) is used in which $R'_1$ represents a methyl radical.

9. Process according to claim 2, characterized in that any one of the products of formula ($I_1$) are prepared, the names of which follow:
   - 1-methyl-1,2,5,6-tetrahydropyridin-3-carboxaldehyde 0-methyl oxime;
   - 1-ethyl-1,2,5,6-tetrahydropyridin-3-carboxaldehyde 0-methyl oxime;
   - 1,2,5,6-tetrahydropyridin-3-carboxaldehyde 0-methyl oxime;
   - 1-propyl-1,2,5,6-tetrahydropyridin-3-carboxaldehyde 0-methyl oxime;
   - 1-allyl-1,2,5,6-tetrahydropyridin-3-carboxaldehyde 0-methyl oxime;

   as well as their salts and notably their hydrochlorides.

**Claims for the following Contracting State : ES**

1. Process for preparing the compounds of formula (I):

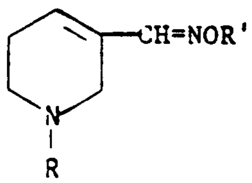

(I)

in which R represents a hydrogen atom, a saturated or unsaturated, linear, branched or cyclic alkyl radical containing up to 8 carbon atoms, optionally substituted by a methoxycarbonyl, ethoxycarbonyl, linear or branched propoxycarbonyl or linear or branched butoxycarbonyl radical, or R represents an aralkyl radical containing up to 10 carbon atoms and R' represents a saturated or unsaturated, linear or branched alkyl radical containing up to 8 carbon atoms, a $-COalk_1$ radical or a $-(CH_2)_2N(alk_2)_2$ radical, $alk_1$ and $alk_2$ representing an alkyl radical containing up to 8 carbon atoms, as well as their addition salts with acids,
characterized in that a compound of formula (II):

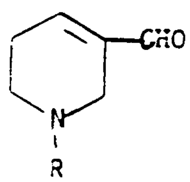

(II)

or one of its salts,
in which R keeps the same meaning as previously, is subjected to the action of a compound of formula (III):

$NH_2$-$OR'_1$ (III)

or one of its salts,
in which $R'_1$ represents a hydrogen atom or a saturated or unsaturated, linear or branched alkyl radical containing up to 8 carbon atoms, in order to obtain the corresponding compound of formula ($I_A$):

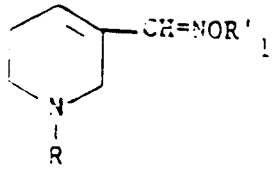

($I_A$)

which if appropriate is salified, or is subjected, in the case where $R'_1$ represents a hydrogen atom, to the action of a compound of formula (IV):

$R'_2$Hal (IV)

$R'_2$ representing a $COalk_1$ radical or a $-(CH_2)_2N-(alk_2)_2$ radical, $alk_1$ and $alk_2$ being defined as previously and Hal representing a halogen atom, in order to obtain the corresponding compound of formula ($I_B$):

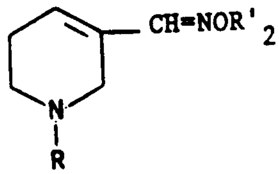

($I_B$)

which is appropriate is salified, in which compound of formula ($I_A$) $R'_1$ does not represent a hydrogen atom or which compound of formula ($I_B$) is subjected, if appropriate, in the case where R represents a hydrogen atom, to the action of a compound of formula (VI):

$R_1Hal$ (IV)

in which Hal represents a halogen atom and $R_1$ represents a saturated or unsaturated, linear, branched or cyclic alkyl radical containing up to 8 carbon atoms, optionally substituted by an esterified carboxy radical or $R_1$ represents an aralkyl radical containing up to 10 carbon atoms, in order to obtain a compound of formula ($I_C$):

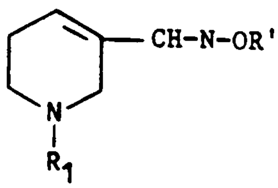

($I_C$)

in which $R_1$ and R' have the meaning indicated previously, which if appropriate is salified, or is subjected, in the case where $R_1$ represents an alkyl radical substituted by an esterified carboxy radical, to the action of a hydrolysis agent in order to obtain the corresponding compound comprising a free carboxy radical, which compound, if appropriate, is salified.

**2.** Process according to claim 1, for preparing the products corresponding to formula ($I_1$):

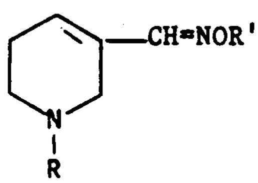

($I_1$)

in which R represents a hydrogen atom or a saturated or unsaturated, linear or branched alkyl radical containing up to 8 carbon atoms and R' has the meaning already indicated, as well as their addition salts with acids, characterized in that a compound of formula (II):

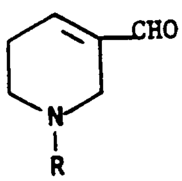

(II)

or one of its salts,
in which R keeps the same meaning as previously, is subjected to the action of a compound of formula

(III):

$NH_2OR'_1$ (III)

or one of its salts,
in which $R'_1$ represents a hydrogen atom or a saturated or unsaturated, linear or branched alkyl radical containing up to 8 carbon atoms, in order to obtain the corresponding compound of formula ($I_A$):

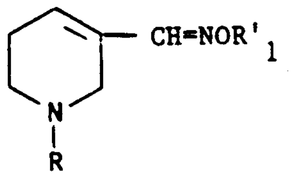

($I_A$)

which if appropriate is salified, or is subjected, in the case where $R'_1$ represents a hydrogen atom, to the action of a compound of formula (IV):

$R'_2Hal$ (IV)

$R'_2$ representing a $COalk_1$ radical or a $-(CH_2)_2N-(alk_2)_2$ radical, $alk_1$ and $alk_2$ being defined as previously and Hal representing a halogen atom, in order to obtain a corresponding compound of formula ($I_B$):

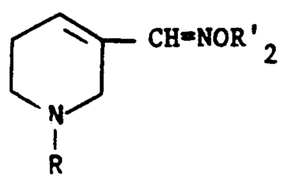

($I_B$)

which if appropriate is salified.

**3.** Variant of the process according to claim 1, characterized in that a compound of formula (V):

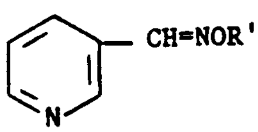

(V)

in which R' keeps the same meaning as in claim 1, is subjected to the action of a compound of formula (VI) as defined in claim 1, in order to obtain the compound of formula (VII):

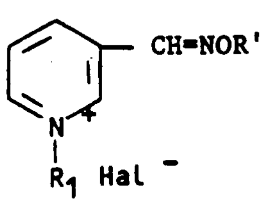

(VII)

which is subjected to the action of a hydrogenation agent, in order to obtain the corresponding compound of formula ($I_C$):

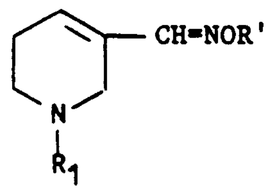

$(I_C)$

in which $R_1$ and R' have the meaning indicated previously, which if appropriate is salified, or is subjected, in the case where $R_1$ represents an alkyl radical substituted by an esterified carboxy radical, to the action of a hydrolysis agent in order to obtain the corresponding compound containing a free carboxy radical, which compound, if appropriate, is salified.

**4.** Variant of the process according to claim 2, characterized in that a compound of formula (V):

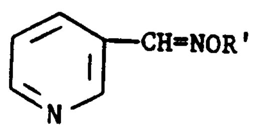

(V)

in which R' keeps the same meaning as in claim 1, is subjected to the action of a compound of formula (VI) as defined in claim 1, in order to obtain the compound of formula (VII):

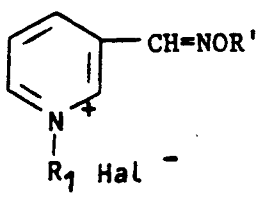

(VII)

which is subjected to the action of a hydrogenation agent, in order to obtain the corresponding compound of formula ($I_C$):

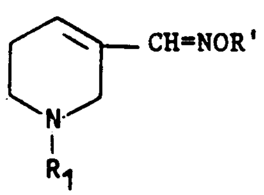

$(I_C)$

which, if appropriate, is salified.

**5.** Process according to claim 2, characterized in that at the start a compound of formula (II) is used in which R represents a hydrogen atom.

**6.** Process according to claim 2, characterized in that at the start a compound of formula (II) is used in which R represents a saturated or unsaturated alkyl radical containing 1 to 4 carbon atoms.

**7.** Process according to claim 2, characterized in that at the start a compound of formula (II) is used in which R represents a methyl, ethyl, propyl or allyl radical.

**8.** Process according to claim 2, characterized in that at the start a compound of formula (III) is used in which $R'_1$ represents a methyl radical.

9. Process according to claim 2, characterized in that any one of the products of formula ($I_1$) are prepared, the names of which follow:
   - 1-methyl-1,2,5,6-tetrahydropyridin-3-carboxaldehyde 0-methyl oxime;
   - 1-ethyl-1,2,5,6-tetrahydropyridin-3-carboxaldehyde 0-methyl oxime;
   - 1,2,5,6-tetrahydropyridin-3-carboxaldehyde 0-methyl oxime;
   - 1-propyl-1,2,5,6-tetrahydropyridin-3-carboxaldehyde 0-methyl oxime;
   - 1-allyl-1,2,5,6-tetrahydropyridin-3-carboxaldehyde 0-methyl oxime;

   as well as their salts and notably their hydrochlorides.

**Claims for the following Contracting State : GR**

1. Process for preparing the compounds of formula (I):

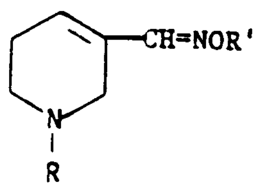

(I)

in which R represents a hydrogen atom, a saturated or unsaturated, linear, branched or cyclic alkyl radical containing up to 8 carbon atoms, optionally substituted by a methoxycarbonyl, ethoxycarbonyl, linear or branched propoxycarbonyl or linear or branched butoxycarbonyl radical, or R represents an aralkyl radical containing up to 10 carbon atoms and R' represents a saturated or unsaturated, linear or branched alkyl radical containing up to 8 carbon atoms, a -COalk$_1$ radical or a $-(CH_2)_2N(alk_2)_2$ radical, alk$_1$ and alk$_2$ representing an alkyl radical containing up to 8 carbon atoms, as well as their addition salts with acids,
characterized in that a compound of formula (II):

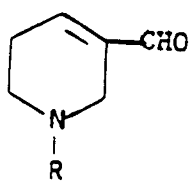

(II)

or one of its salts,
in which R keeps the same meaning as previously, is subjected to the action of a compound of formula (III):

$NH_2$-OR'$_1$ (III)

or one of its salts,
in which R'1 represents a hydrogen atom or a saturated or unsaturated, linear or branched alkyl radical containing up to 8 carbon atoms, in order to obtain the corresponding compound of formula ($I_A$):

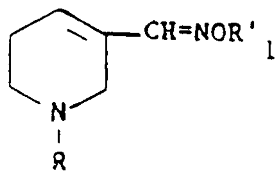

($I_A$)

which if appropriate is salified, or is subjected, in the case where R'$_1$ represents a hydrogen atom, to the action of a compound of formula (IV):

R'$_2$Hal (IV)

$R'_2$ representing a $COalk_1$ radical or a $-(CH_2)_2N-(alk_2)_2$ radical, $alk_1$ and $alk_2$ being defined as previously and Hal representing a halogen atom, in order to obtain the corresponding compound of formula ($I_B$):

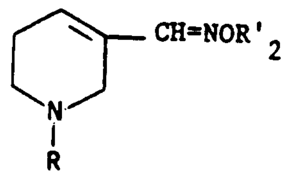

($I_B$)

which is appropriate is salified, in which compound of formula ($I_A$) R'1 does not represent a hydrogen atom or which compound of formula ($I_B$) is subjected, if appropriate, in the case where R represents a hydrogen atom, to the action of a compound of formula (VI):

$R_1$Hal (IV)

in which Hal represents a halogen atom and $R_1$ represents a saturated or unsaturated, linear, branched or cyclic alkyl radical containing up to 8 carbon atoms, optionally substituted by an esterified carboxy radical or $R_1$ represents an aralkyl radical containing up to 10 carbon atoms, in order to obtain a compound of formula ($I_C$):

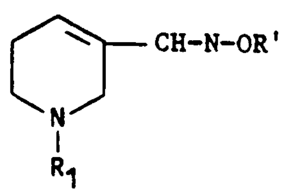

($I_C$)

in which $R_1$ and R' have the meaning indicated previously, which if appropriate is salified, or is subjected, in the case where $R_1$ represents an alkyl radical substituted by an esterified carboxy radical, to the action of a hydrolysis agent in order to obtain the corresponding compound comprising a free carboxy radical, which compound, if appropriate, is salified.

**2.** Process according to claim 1, for preparing the products corresponding to formula ($I_1$):

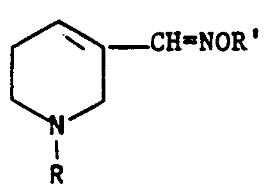

($I_1$)

in which R represents a hydrogen atom or a saturated or unsaturated, linear or branched alkyl radical containing up to 8 carbon atoms and R' has the meaning already indicated, as well as their addition salts with acids, characterized in that a compound of formula (II):

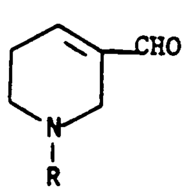

(II)

or one of its salts,
in which R keeps the same meaning as previously, is subjected to the action of a compound of formula

(III):

$NH_2OR'_1$ (III)

or one of its salts,
in which $R'_1$ represents a hydrogen atom or a saturated or unsaturated, linear or branched alkyl radical containing up to 8 carbon atoms, in order to obtain the corresponding compound of formula ($I_A$):

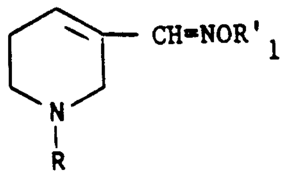

($I_A$)

which if appropriate is salified, or is subjected, in the case where $R'_1$ represents a hydrogen atom, to the action of a compound of formula (IV):

$R'_2Hal$ (IV)

$R'_2$ representing a $COalk_1$ radical or a $-(CH_2)_2N-(alk_2)_2$ radical, $alk_1$ and $alk_2$ being defined as previously and Hal representing a halogen atom, in order to obtain the corresponding compound of formula ($I_B$):

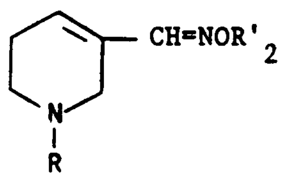

($I_B$)

which if appropriate is salified.

**3.** Variant of the process according to claim 1, characterized in that a compound of formula (V):

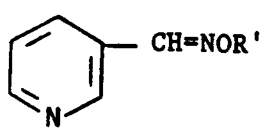

(V)

in which R' keeps the same meaning as in claim 1, is subjected to the action of a compound of formula (VI) as defined in claim 1, in order to obtain the compound of formula (VII):

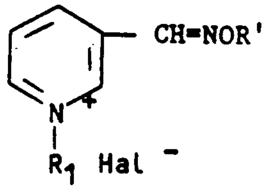

(VII)

which is subjected to the action of a hydrogenation agent, in order to obtain the corresponding compound of formula ($I_C$):

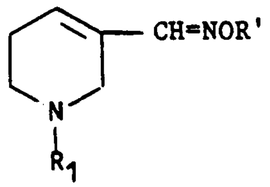

(I$_C$)

in which $R_1$ and R' have the meaning indicated previously, which if appropriate is salified, or is subjected, in the case where $R_1$ represents an alkyl radical substituted by an esterified carboxy radical, to the action of a hydrolysis agent in order to obtain the corresponding compound containing a free carboxy radical, which compound, if appropriate, is salified.

4. Variant of the process according to claim 2, characterized in that a compound of formula (V):

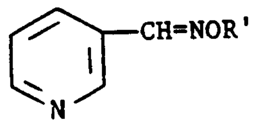

(V)

in which R' keeps the same meaning as in claim 1, is subjected to the action of a compound of formula (VI) as defined in claim 1, in order to obtain the compound of formula (VII):

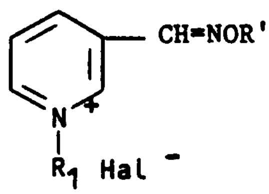

(VII)

which is subjected to the action of a hydrogenation agent, in order to obtain the corresponding compound of formula ($I_C$):

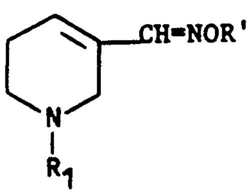

(I$_C$)

which, if appropriate, is salified.

5. Process according to claim 2, characterized in that at the start a compound of formula (II) is used in which R represents a hydrogen atom.

6. Process according to claim 2, characterized in that at the start a compound of formula (II) is used in which R represents a saturated or unsaturated alkyl radical containing 1 to 4 carbon atoms.

7. Process according to claim 2, characterized in that at the start a compound of formula (II) is used in which R represents a methyl, ethyl, propyl or allyl radical.

8. Process according to claim 2, characterized in that at the start a compound of formula (III) is used in which $R'_1$ represents a methyl radical.

**9.** Process according to claim 2, characterized in that any one of the products of formula ($I_1$) are prepared, the names of which follow:
- 1-methyl-1,2,5,6-tetrahydropyridin-3-carboxaldehyde 0-methyl oxime;
- 1-ethyl-1,2,5,6-tetrahydropyridin-3-carboxaldehyde 0-methyl oxime;
- 1,2,5,6-tetrahydropyridin-3-carboxaldehyde 0-methyl oxime;
- 1-propyl-1,2,5,6-tetrahydropyridin-3-carboxaldehyde 0-methyl oxime;
- 1-allyl-1,2,5,6-tetrahydropyridin-3-carboxaldehyde 0-methyl oxime;

as well as their salts and notably their hydrochlorides.

**10.** Preparation process for pharmaceutical compositions, characterized in that at least one of the products of formula (I), as defined in claim 1, is used as active ingredient in a form intended for this use.

**11.** Preparation process for pharmaceutical compositions, characterized in that at least one of the products of formula ($I_1$), as defined in claim 2, is used as active ingredient in a form intended for this use.

**12.** Preparation process for pharmaceutical compositions, characterized in that at least one of the products of formula (I), as defined in claim 9, is used as active ingredient in a form intended for this use.

**Patentansprüche**

**Patentansprüche für folgende Vertragsstaaten : BE, CH, DE, FR, GB, IT, LI, LU, NL, SE**

**1.** Verbindungen der Formel (I)

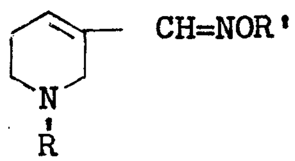

(I)

worin R für ein Wasserstoffatom, einen gesättigten oder ungesättigten, linearen, verzweigten oder cyclischen Alkylrest mit bis zu 8 Kohlenstoffatomen, der gegebenenfalls durch einen Methoxycarbonyl-, Ethoxycarbonyl-, linearen oder verzweigten Propoxycarbonyl- oder linearen oder verzweigten Butoxycarbonylrest substituiert ist, steht oder R einen Aralkylrest mit bis zu 10 Kohlenstoffatomen bedeutet und R' einen gesättigten oder ungesättigten, linearen oder verzweigten Alkylrest mit bis zu 8 Kohlenstoffatomen, einen Rest $-COalc_1$ oder einen Rest $-(CH_2)_2N(alc_2)_2$ bedeutet, wobei $alc_1$ und $alc_2$ einen Alkylrest mit bis zu 8 Kohlenstoffatomen wiedergeben, sowie deren Additionssalze mit Säuren.

**2.** Verbindungen der Formel (I) gemäß Anspruch 1, worin R für ein Wasserstoffatom steht, sowie deren Additionssalze mit Säuren.

**3.** Verbindungen der Formel (I) gemäß Anspruch 1, worin R für einen gesättigten oder ungesättigten Alkylrest mit 1 bis 4 Kohlenstoffatomen steht, sowie deren Additionssalze mit Säuren.

**4.** Verbindungen der Formel (I) gemäß Anspruch 3, worin R für einen Methyl-, Ethyl-, Propyl- oder Allylrest steht, sowie deren Additionssalze mit Säuren.

**5.** Verbindungen der Formel (I) gemäß Anspruch 1 bis 4, worin R' für einen Methylrest steht, sowie deren Additionssalze mit Säuren.

**6.** Verbindungen der Formel, wie in Anspruch 1 definiert, mit den folgenden Bezeichnungen:

1-Methyl-1,2,5,6-tetrahydropyridin-3-carboxaldehyd-0-methyloxim;
1-Ethyl-1,2,5,6-tetrahydropyridin-3-carboxaldehyd-0-methyloxim;
1,2,5,6-Tetrahydropyridin-3-carboxaldehyd-0-methyloxim;
1-Propyl-1,2,5,6-tetrahydropyridin-3-carboxaldehyd-0-methyloxim;
1-Allyl-1,2,5,6-tetrahydropyridin-3-carboxaldehyd-0-methyloxim;

sowie deren Salze und insbesondere deren Hydrochloride.

**7.** Als Arzneimittel die Verbindungen der Formel (I) gemäß einem der Ansprüche 1 bis 5 sowie deren Additionssalze mit pharmazeutisch verträglichen Säuren.

**8.** Als Arzneimittel die Verbindungen gemäß Anspruch 6 sowie deren Additionssalze mit pharmazeutisch verträglichen Säuren.

**9.** Pharmazeutische Zusammensetzungen, enthaltend als Wirkstoff zumindest eines der Arzneimittel gemäß Anspruch 7 oder 8.

**10.** Verfahren zur Herstellung der Verbindungen der Formel (I) gemäß Anspruch 1, dadurch gekennzeichnet, daß man eine Verbindung der Formel (II)

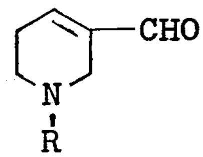

(II)

oder eines ihrer Salze, worin R wie in Anspruch 1 definiert ist, der Einwirkung einer Verbindung der Formel (III)

$NH_2OR'_1$ (III)

oder eines ihrer Salze, worin $R'_1$ für ein Wasserstoffatom oder einen gesättigten oder ungesättigten, linearen oder verzweigten Alkylrest mit bis zu 8 Kohlenstoffatomen steht, unterzieht, um zu der entsprechenden Verbindung der Formel ($I_A$)

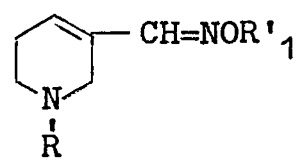

($I_A$)

zu gelangen, die man gegebenenfalls in ein Salz überführt oder die man, wenn $R'_1$ für ein Wasserstoffatom steht, der Einwirkung einer Verbindung der Formel (IV)

$R'_2Hal$ (IV)

unterwirft, worin $R'_2$ einen Rest $COalc_1$ oder einen Rest $-(CH_2)_2N-(alc_2)_2$ bedeutet, wobei $alc_1$ und $alc_2$ wie vorstehend definiert sind und Hal für ein Halogenatom steht, um zu der entsprechenden Verbindung der Formel ($I_B$)

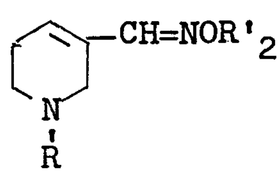

($I_B$)

zu gelangen, die man gegebenenfalls in ein Salz überführt, die Verbindung der Formel ($I_A$), worin $R'_1$ kein Wasserstoffatom bedeutet, oder die Verbindung der Formel ($I_B$) gegebenenfalls, wenn R für ein Wasserstoffatom steht, der Einwirkung einer Verbindung der Formel (VI)

$R_1Hal$ (VI)

unterzieht, worin Hal ein Halogenatom bedeutet und $R_1$ einen gesättigten oder ungesättigten, linearen,

verzweigten oder cyclischen Alkylrest mit bis zu 8 Kohlenstoffatomen wiedergibt, der gegebenenfalls durch einen veresterten Carboxyrest substituiert ist, oder $R_1$ für einen Aralkylrest mit bis zu 10 Kohlenstoffatomen steht, um zu einer Verbindung der Formel ($I_C$)

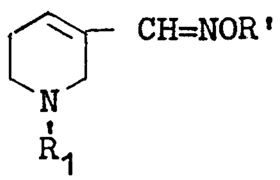

($I_C$)

zu gelangen, worin $R_1$ und R' wie vorstehend definiert sind, die man gegebenenfalls in ein Salz überführt oder die man, wenn $R_1$ einen durch einen veresterten Carboxyrest substituierten Alkylrest wiedergibt, der Einwirkung eines Hydrolysemittels unterzieht, um die entsprechende Verbindung zu erhalten, die eine freie Carboxygruppe aufweist, welche Verbindung man gegebenenfalls in ein Salz überführt.

**11.** Abänderung des Verfahrens gemäß Anspruch 10, dadurch gekennzeichnet, daß man eine Verbindung der Formel (V)

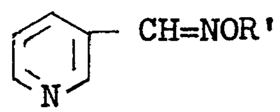

(V)

worin R' die in Anspruch 1 angegebene Bedeutung besitzt, der Einwirkung einer Verbindung der Formel (VI), wie in Anspruch 10 definiert, unterzieht, um zu der Verbindung der Formel (VII)

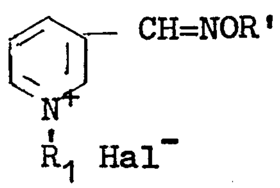

(VII)

zu gelangen, die man der Einwirkung eines Hydrierungsmittels unterzieht, um die entsprechende Verbindung der Formel ($I_C$)

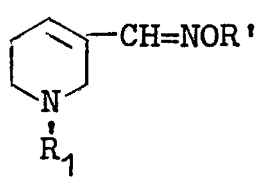

($I_C$)

zu erhalten, worin $R_1$ und R' die vorstehend angegebene Bedeutung besitzen, die man gegebenenfalls in ein Salz überführt oder, wenn $R_1$ einen durch einen veresterten Carboxyrest substituierten Alkylrest bedeutet, der Einwirkung eines Hydrolysemittels unterzieht, um die entsprechende Verbindung zu erhalten, die eine freie Carboxygruppe aufweist, welche Verbindung man gegebenenfalls in ein Salz überführt.

**Patentansprüche für folgenden Vertragsstaat : AT**

**1.** Verfahren zur Herstellung der Verbindungen der Formel (I)

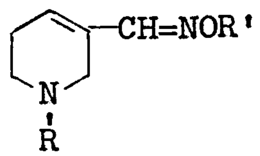

(I)

worin R für ein Wasserstoffatom, einen gesättigten oder ungesättigten, linearen, verzweigten oder cyclischen Alkylrest mit bis zu 8 Kohlenstoffatomen, der gegebenenfalls durch einen Methoxycarbonyl-, Ethoxycarbonyl-, linearen oder verzweigten Propoxycarbonyl- oder linearen oder verzweigten Butoxycarbonylrest substituiert ist, steht oder R einen Aralkylrest mit bis zu 10 Kohlenstoffatomen bedeutet und R' einen gesättigten oder ungesättigten, linearen oder verzweigten Alkylrest mit bis zu 8 Kohlenstoffatomen, einen Rest $-COalc_1$ oder einen Rest $-(CH_2)_2N(alc_2)_2$ bedeutet, wobei $alc_1$ und $alc_2$ einen Alkylrest mit bis zu 8 Kohlenstoffatomen wiedergeben, sowie von deren Additionssalzen mit Säuren,
dadurch gekennzeichnet, daß man eine Verbindung der Formel (II)

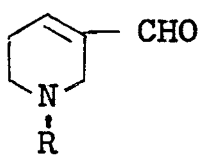

(II)

oder eines ihrer Salze,
worin R die vorstehend angegebene Bedeutung besitzt, der Einwirkung einer Verbindung der Formel (III)

$NH_2OR'_1$ (III)

oder eines ihrer Salze,
worin $R'_1$ für ein Wasserstoffatom oder einen gesättigten oder ungesättigten, linearen oder verzweigten Alkylrest mit bis zu 8 Kohlenstoffatomen steht, unterzieht, um zu der entsprechenden Verbindung der Formel ($I_A$)

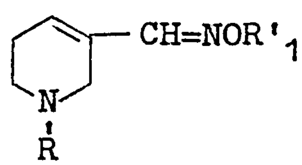

($I_A$)

zu gelangen, die man gegebenenfalls in ein Salz überführt oder die man, wenn $R'_1$ für ein Wasserstoffatom steht, der Einwirkung einer Verbindung der Formel (IV)

$R'_2Hal$ (IV)

unterwirft, worin $R'_2$ einen Rest $COalc_1$ oder einen Rest $-(CH_2)_2N-(alc_2)_2$ bedeutet, wobei $alc_1$ und $alc_2$ wie vorstehend definiert sind und Hal für ein Halogenatom steht, um zu der entsprechenden Verbindung der Formel ($I_B$)

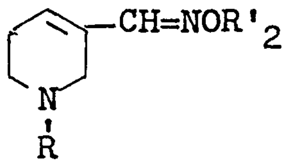

($I_B$)

zu gelangen, die man gegebenenfalls in ein Salz überführt, die Verbindung der Formel ($I_A$), worin $R'_1$ kein Wasserstoffatom bedeutet, oder die Verbindung der Formel ($I_B$) gegebenenfalls, wenn R für ein Wasserstoffatom steht, der Einwirkung einer Verbindung der Formel (VI)

$R_1$ Hal (VI)

unterzieht, worin Hal ein Halogenatom bedeutet und $R_1$ einen gesättigten oder ungesättigten, linearen, verzweigten oder cyclischen Alkylrest mit bis zu 8 Kohlenstoffatomen wiedergibt, der gegebenenfalls durch einen veresterten Carboxyrest substituiert ist, oder $R_1$ für einen Aralkylrest mit bis zu 10 Kohlenstoffatomen steht, um zu einer Verbindung der Formel ($I_C$)

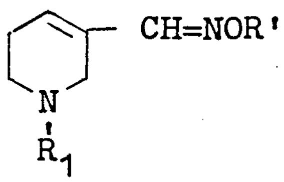

($I_C$)

zu gelangen, worin $R_1$ und R' wie vorstehend definiert sind, die man gegebenenfalls in ein Salz überführt oder die man, wenn $R_1$ einen durch einen veresterten Carboxyrest substituierten Alkylrest wiedergibt, der Einwirkung eines Hydrolysemittels unterzieht, um die entsprechende Verbindung zu erhalten, die eine freie Carboxygruppe aufweist, welche Verbindung man gegebenenfalls in ein Salz überführt.

**2.** Verfahren gemäß Anspruch 1 zur Herstellung der der Formel ($I_1$)

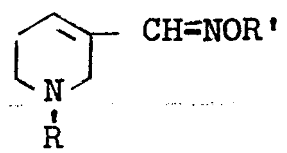

($I_1$)

entsprechenden Produkte, worin R für ein Wasserstoffatom oder einen gesättigten oder ungesättigten, linearen oder verzweigten Alkylrest mit bis zu 8 Kohlenstoffatomen steht und R' die angegebene Bedeutung hat, sowie von deren Additionssalzen mit Säuren, dadurch gekennzeichnet, daß man eine Verbindung der Formel (II)

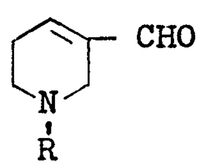

(II)

oder eines ihrer Salze, worin R wie vorstehend definiert ist, der Einwirkung einer Verbindung der Formel (III)

$NH_2OR'_1$ (III)

oder eines ihrer Salze, worin $R'_1$ für ein Wasserstoffatom oder einen gesättigten oder ungesättigten, linearen oder verzweigten Alkylrest mit bis zu 8 Kohlenstoffatomen steht, unterzieht, um zu der

entsprechenden Verbindung der Formel ($I_A$)

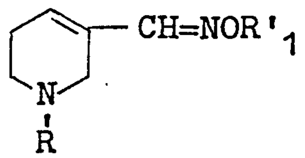

($I_A$)

zu gelangen, die man gegebenenfalls in ein Salz überführt oder die man, wenn $R'_1$ für ein Wasserstoffatom steht, der Einwirkung einer Verbindung der Formel (IV)

$R'_2Hal$ (IV)

unterwirft, worin $R'_2$ einen Rest $COalc_1$ oder einen Rest $-(CH_2)_2N-(alc_2)_2$ bedeutet, wobei $alc_1$ und $alc_2$ wie vorstehend definiert sind und Hal für ein Halogenatom steht, um zu der entsprechenden Verbindung der Formel ($I_B$)

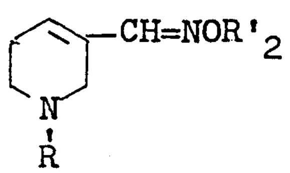

($I_B$)

zu gelangen, die man gegebenenfalls in ein Salz überführt.

**3.** Abänderung des Verfahrens gemäß Anspruch 1, dadurch gekennzeichnet, daß man eine Verbindung der Formel (V)

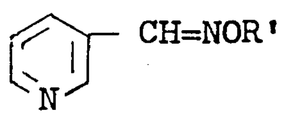

(V)

worin R' die in Anspruch 1 angegebene Bedeutung besitzt, der Einwirkung einer Verbindung der Formel (VI), wie in Anspruch 1 definiert, unterzieht, um zu der Verbindung der Formel (VII)

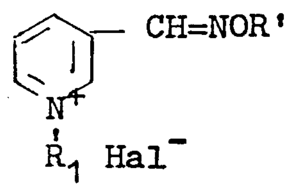

(VII)

zu gelangen, die man der Einwirkung eines Hydrierungsmittels unterzieht, um die entsprechende Verbindung der Formel ($I_C$)

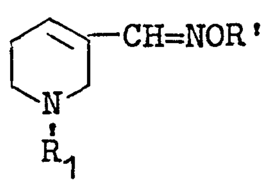

($I_C$)

zu erhalten, worin $R_1$ und R' die vorstehend angegebene Bedeutung besitzen, die man gegebenenfalls in ein Salz überführt oder, wenn $R_1$ einen durch einen veresterten Carboxyrest substituierten Alkylrest

bedeutet, der Einwirkung eines Hydrolysemittels unterzieht, um die entsprechende Verbindung zu erhalten, die eine freie Carboxygruppe aufweist, welche Verbindung man gegebenenfalls in ein Salz überführt.

**4.** Abänderung des Verfahrens gemäß Anspruch 2, dadurch gekennzeichnet, daß man eine Verbindung der Formel (V)

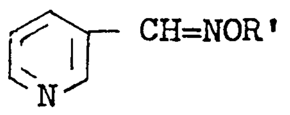

(V)

worin R' die in Anspruch 1 angegebene Bedeutung besitzt, der Einwirkung einer Verbindung der Formel (VI), wie in Anspruch 1 definiert, unterzieht, um zu der Verbindung der Formel (VII)

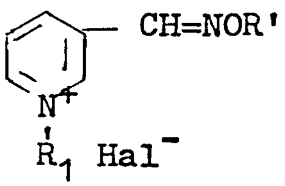

(VII)

zu gelangen, die man der Einwirkung eines Hydrierungsmittels unterzieht, um die entsprechende Verbindung der Formel ($I_C$)

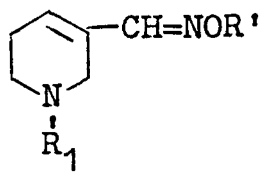

($I_C$)

zu erhalten, die man gegebenenfalls in ein Salz überführt.

**5.** Verfahren gemäß Anspruch 2, dadurch gekennzeichnet, daß man von einer Verbindung der Formel (II) ausgeht, worin R ein Wasserstoffatom bedeutet.

**6.** Verfahren gemäß Anspruch 2, dadurch gekennzeichnet, daß man von einer Verbindung der Formel (II) ausgeht, worin R einen gesättigten oder ungesättigten Alkylrest mit 1 bis 4 Kohlenstoffatomen bedeutet.

**7.** Verfahren gemäß Anspruch 2, dadurch gekennzeichnet, daß man von einer Verbindung der Formel (II) ausgeht, worin R einen Methyl-, Ethyl-, Propyl- oder Allylrest bedeutet.

**8.** Verfahren gemäß Anspruch 2, dadurch gekennzeichnet, daß man von einer Verbindung der Formel (III) ausgeht, worin $R'_1$ einen Methylrest bedeutet.

**9.** Verfahren gemäß Anspruch 2, dadurch gekennzeichnet, daß man eines der Produkte der Formel ($I_1$) mit den folgenden Bezeichnungen herstellt:
1-Methyl-1,2,5,6-tetrahydropyridin-3-carboxaldehyd-0-methyloxim;
1-Ethyl-1,2,5,6-tetrahydropyridin-3-carboxaldehyd-0-methyl-oxim;
1,2,5,6-Tetrahydropyridin-3-carboxaldehyd-0-methyloxim;
1-Propyl-1,2,5,6-tetrahydropyridin-3-carboxaldehyd-0-methyloxim;
1-Allyl-1,2,5,6-tetrahydropidin-3-carboxaldehyd-0-methyloxim;
sowie deren Salze und insbesondere deren Hydrochloride.

**Patentansprüche für folgenden Vertragsstaat : ES**

**1.** Verfahren zur Herstellung der Verbindungen der Formel (I)

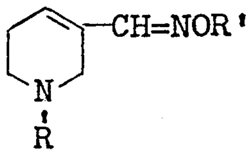

(I)

worin R für ein Wasserstoffatom, einen gesättigten oder ungesättigten, linearen, verzweigten oder cyclischen Alkylrest mit bis zu 8 Kohlenstoffatomen, der gegebenenfalls durch einen Methoxycarbonyl-, Ethoxycarbonyl-, linearen oder verzweigten Propoxycarbonyl- oder linearen oder verzweigten Butoxycarbonylrest substituiert ist, steht oder R einen Aralkylrest mit bis zu 10 Kohlenstoffatomen bedeutet und R' einen gesättigten oder ungesättigten, linearen oder verzweigten Alkylrest mit bis zu 8 Kohlenstoffatomen, einen Rest $-COalc_1$ oder einen Rest $-(CH_2)_2N(alc_2)_2$ bedeutet, wobei $alc_1$ und $alc_2$ einen Alkylrest mit bis zu 8 Kohlenstoffatomen wiedergeben, sowie von deren Additionssalzen mit Säuren,

dadurch gekennzeichnet, daß man eine Verbindung der Formel (II)

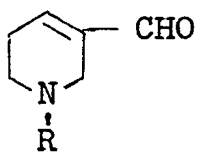

(II)

oder eines ihrer Salze,

worin R die vorstehend angegebene Bedeutung besitzt, der Einwirkung einer Verbindung der Formel (III)

$NH_2OR'_1$ (III)

oder eines ihrer Salze,

worin $R'_1$ für ein Wasserstoffatom oder einen gesättigten oder ungesättigten, linearen oder verzweigten Alkylrest mit bis zu 8 Kohlenstoffatomen steht, unterzieht, um zu der entsprechenden Verbindung der Formel ($I_A$)

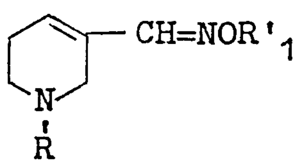

($I_A$)

zu gelangen, die man gegebenenfalls in ein Salz überführt oder die man, wenn $R'_1$ für ein Wasserstoffatom steht, der Einwirkung einer Verbindung der Formel (IV)

$R'_2Hal$ (IV)

unterwirft, worin $R'_2$ einen Rest $COalc_1$ oder einen Rest $-(CH_2)_2N-(alc_2)_2$ bedeutet, wobei $alc_1$ und $alc_2$ wie vorstehend definiert sind und Hal für ein Halogenatom steht, um zu der entsprechenden Verbindung der Formel ($I_B$)

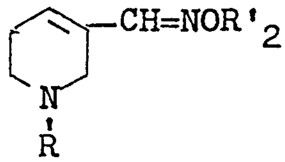

($I_B$)

zu gelangen, die man gegebenenfalls in ein Salz überführt, die Verbindung der Formel ($I_A$), worin $R'_1$ kein Wasserstoffatom bedeutet,oder die Verbindung der Formel ($I_B$) gegebenenfalls, wenn R für ein Wasserstoffatom steht, der Einwirkung einer Verbindung der Formel (VI)

$R_1$Hal (IV)

unterzieht, worin Hal ein Halogenatom bedeutet und $R_1$ einen gesättigten oder ungesättigten, linearen, verzweigten oder cyclischen Alkylrest mit bis zu 8 Kohlenstoffatomen wiedergibt, der gegebenenfalls durch einen veresterten Carboxyrest substituiert ist, oder $R_1$ für einen Aralkylrest mit bis zu 10 Kohlenstoffatomen steht, um zu einer Verbindung der Formel ($I_C$)

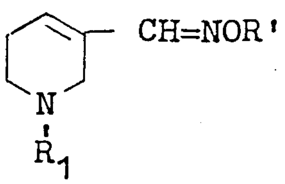

($I_C$)

zu gelangen, worin $R_1$ und R' wie vorstehend definiert sind, die man gegebenenfalls in ein Salz überführt oder die man, wenn $R_1$ einen durch einen veresterten Carboxyrest substituierten Alkylrest wiedergibt, der Einwirkung eines Hydrolysemittels unterzieht, um die entsprechende Verbindung zu erhalten, die eine freie Carboxygruppe aufweist, welche Verbindung man gegebenenfalls in ein Salz überführt.

**2.** Verfahren gemäß Anspruch 1 zur Herstellung der der Formel ($I_1$)

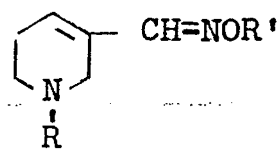

($I_1$)

entsprechenden Produkte, worin R für ein Wasserstoffatom oder einen gesättigten oder ungesättigten, linearen oder verzweigten Alkylrest mit bis zu 8 Kohlenstoffatomen steht und R' die angegebene Bedeutung hat, sowie von deren Additionssalzen mit Säuren, dadurch gekennzeichnet, daß man eine Verbindung der Formel (II)

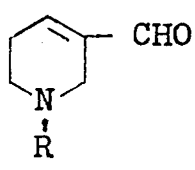

(II)

oder eines ihrer Salze, worin R wie vorstehend definiert ist, der Einwirkung einer Verbindung der Formel (III)

$NH_2OR'_1$ (III)

oder eines ihrer Salze, worin $R'_1$ für ein Wasserstoffatom oder einen gesättigten oder ungesättigten, linearen oder verzweigten Alkylrest mit bis zu 8 Kohlenstoffatomen steht, unterzieht, um zu der entsprechenden Verbindung der Formel ($I_A$)

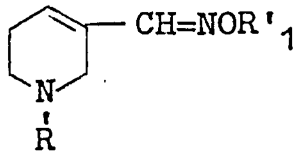

$(I_A)$

zu gelangen, die man gegebenenfalls in ein Salz überführt oder die man, wenn $R'_1$ für ein Wasserstoffatom steht, der Einwirkung einer Verbindung der Formel (IV)

$R'_2Hal$ (IV)

unterwirft, worin $R'_2$ einen Rest $COalc_1$ oder einen Rest $-(CH_2)_2N-(alc_2)_2$ bedeutet, wobei $alc_1$ und $alc_2$ wie vorstehend definiert sind und Hal für ein Halogenatom steht, um zu der entsprechenden Verbindung der Formel $(I_B)$

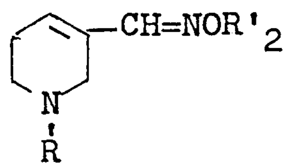

$(I_B)$

zu gelangen, die man gegebenenfalls in ein Salz überführt.

**3.** Abänderung des Verfahrens gemäß Anspruch 1, dadurch gekennzeichnet, daß man eine Verbindung der Formel (V)

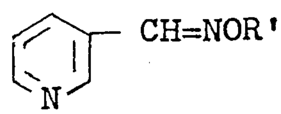

(V)

worin R' die in Anspruch 1 angegebene Bedeutung besitzt, der Einwirkung einer Verbindung der Formel (VI), wie in Anspruch 1 definiert, unterzieht, um zu der Verbindung der Formel (VII)

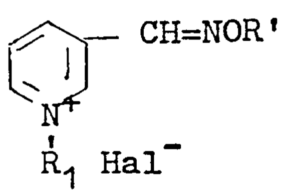

(VII)

zu gelangen, die man der Einwirkung eines Hydrierungsmittels unterzieht, um die entsprechende Verbindung der Formel $(I_C)$

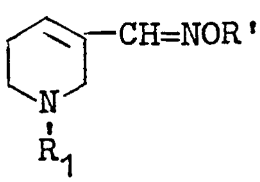

$(I_C)$

zu erhalten, worin $R_1$ und R' die vorstehend angegebene Bedeutung besitzen, die man gegebenenfalls

in ein Salz überführt oder, wenn $R_1$ einen durch einen veresterten Carboxyrest substituierten Alkylrest bedeutet, der Einwirkung eines Hydrolysemittels unterzieht, um die entsprechende Verbindung zu erhalten, die eine freie Carboxygruppe aufweist, welche Verbindung man gegebenenfalls in ein Salz überführt.

**4.** Abänderung des Verfahrens gemäß Anspruch 2, dadurch gekennzeichnet, daß man eine Verbindung der Formel (V)

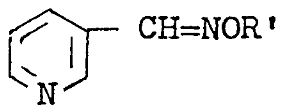

(V)

worin R' die in Anspruch 1 angegebene Bedeutung besitzt, der Einwirkung einer Verbindung der Formel (VI), wie in Anspruch 1 definiert, unterzieht, um zu der Verbindung der Formel (VII)

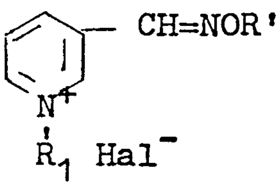

(VII)

zu gelangen, die man der Einwirkung eines Hydrierungsmittels unterzieht, um die entsprechende Verbindung der Formel ($I_C$)

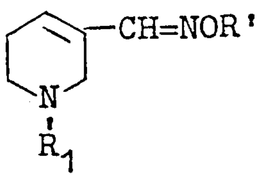

($I_C$)

zu erhalten, die man gegebenenfalls in ein Salz überführt.

**5.** Verfahren gemäß Anspruch 2, dadurch gekennzeichnet, daß man von einer Verbindung der Formel (II) ausgeht, worin R ein Wasserstoffatom bedeutet.

**6.** Verfahren gemäß Anspruch 2, dadurch gekennzeichnet, daß man von einer Verbindung der Formel (II) ausgeht, worin R einen gesättigten oder ungesättigten Alkylrest mit 1 bis 4 Kohlenstoffatomen bedeutet.

**7.** Verfahren gemaß Anspruch 2, dadurch gekennzeichnet, daß man von einer Verbindung der Formel (II) ausgeht, worin R einen Methyl-, Ethyl-, Propyl- oder Allylrest bedeutet.

**8.** Verfahren gemäß Anspruch 2, dadurch gekennzeichnet, daß man von einer Verbindung der Formel (III) ausgeht, worin $R'_1$ einen Methylrest bedeutet.

**9.** Verfahren gemäß Anspruch 2, dadurch gekennzeichnet, daß man eines der Produkte der Formel ($I_1$) mit den folgenden Bezeichnungen herstellt:

1-Methyl-1,2,5,6-tetrahydropyridin-3-carboxaldehyd-0-methyloxim;
1-Ethyl-1,2,5,6-tetrahydropyridin-3-carboxaldehyd-0-methyl-oxim;
1,2,5,6-Tetrahydropyridin-3-carboxaldehyd-0-methyloxim;

1-Propyl-1,2,5,6-tetrahydropyridin-3-carboxaldehyd-0-methyloxim;
1-Allyl-1,2,5,6-tetrahydropidin-3-carboxaldehyd-0-methyloxim;
sowie deren Salze und insbesondere deren Hydrochloride.

**Patentansprüche für folgenden Vertragsstaat : GR**

**1.** Verfahren zur Herstellung der Verbindungen der Formel (I)

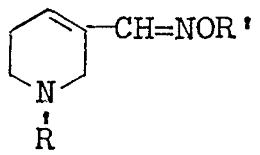

(I)

worin R für ein Wasserstoffatom, einen gesättigten oder ungesättigten, linearen, verzweigten oder cyclischen Alkylrest mit bis zu 8 Kohlenstoffatomen, der gegebenenfalls durch einen Methoxycarbonyl, Ethoxycarbonyl-, linearen oder verzweigten Propoxycarbonyl- oder linearen oder verzweigten Butoxycarbonylrest substituiert ist, steht oder R einen Aralkylrest mit bis zu 10 Kohlenstoffatomen bedeutet und R' einen gesättigten oder ungesättigten, linearen oder verzweigten Alkylrest mit bis zu 8 Kohlenstoffatomen, einen Rest -COalc$_1$ oder einen Rest $-(CH_2)_2N(alc_2)_2$ bedeutet, wobei alc$_1$ und alc$_2$ einen Alkylrest mit bis zu 8 Kohlenstoffatomen wiedergeben, sowie von deren Additionssalzen mit Säuren,
dadurch gekennzeichnet, daß man eine Verbindung der Formel (II)

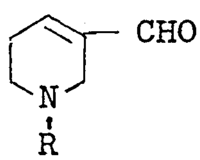

(II)

oder eines ihrer Salze,
worin R die vorstehend angegebene Bedeutung besitzt, der Einwirkung einer Verbindung der Formel (III)

$NH_2OR'_1$ (III)

oder eines ihrer Salze,
worin $R'_1$ für ein Wasserstoffatom oder einen gesättigten oder ungesättigten, linearen oder verzweigten Alkylrest mit bis zu 8 Kohlenstoffatomen steht, unterzieht, um zu der entsprechenden Verbindung der Formel ($I_A$)

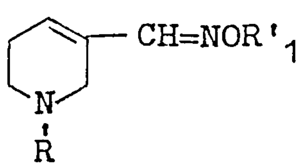

($I_A$)

zu gelangen, die man gegebenenfalls in ein Salz überführt oder die man, wenn $R'_1$ für ein Wasserstoffatom steht, der Einwirkung einer Verbindung der Formel (IV)

$R'_2Hal$ (IV)

unterwirft, worin $R'_2$ einen Rest COalc$_1$ oder einen Rest $-(CH_2)_2N-(alc_2)_2$ bedeutet, wobei alc$_1$ und alc$_2$ wie vorstehend definiert sind und Hai für ein Halogenatom steht, um zu der entsprechenden Verbindung der Formel ($I_B$)

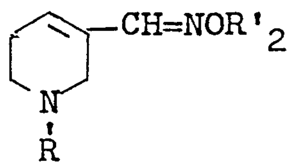

$(I_B)$

zu gelangen, die man gegebenenfalls in ein Salz überführt, die Verbindung der Formel ($I_A$), worin $R'_1$ kein Wasserstoffatom bedeutet, oder die Verbindung der Formel ($I_B$) gegebenenfalls, wenn R für ein Wasserstoffatom steht, der Einwirkung einer Verbindung der Formel (VI)

$R_1 Hal$ (VI)

unterzieht, worin Hal ein Halogenatom bedeutet und $R_1$ einen gesättigten oder ungesättigten, linearen, verzweigten oder cyclischen Alkylrest mit bis zu 8 Kohlenstoffatomen wiedergibt, der gegebenenfalls durch einen veresterten Carboxyrest substituiert ist, oder $R_1$ für einen Aralkylrest mit bis zu 10 Kohlenstoffatomen steht, um zu einer Verbindung der Formel ($I_C$)

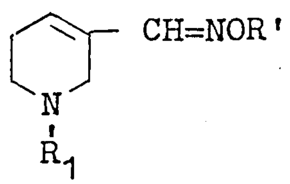

$(I_C)$

zu gelangen, worin $R_1$ und R' wie vorstehend definiert sind, die man gegebenenfalls in ein Salz überführt oder die man, wenn $R_1$ einen durch einen veresterten Carboxyrest substituierten Alkylrest wiedergibt, der Einwirkung eines Hydrolysemittels unterzieht, um die entsprechende Verbindung zu erhalten, die eine freie Carboxygruppe aufweist, welche Verbindung man gegebenenfalls in ein Salz überführt.

**2.** Verfahren gemäß Anspruch 1 zur Herstellung der der Formel ($I_1$)

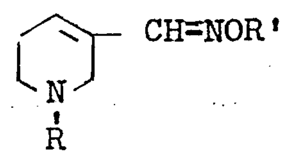

$(I_1)$

entsprechenden Produkte, worin R für ein Wasserstoffatom oder einen gesättigten oder ungesättigten, linearen oder verzweigten Alkylrest mit bis zu 8 Kohlenstoffatomen steht und R' die angegebene Bedeutung hat, sowie von deren Additionssalzen mit Säuren, dadurch gekennzeichnet, daß man eine Verbindung der Formel (II)

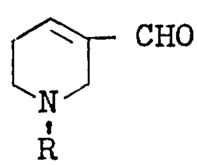

(II)

oder eines ihrer Salze, worin R wie vorstehend definiert ist, der Einwirkung einer Verbindung der Formel (III)

$NH_2 OR'_1$ (III)

oder eines ihrer Salze, worin $R'_1$ für ein Wasserstoffatom oder einen gesättigten oder ungesättigten, linearen oder verzweigten Alkylrest mit bis zu 8 Kohlenstoffatomen steht, unterzieht, um zu der entsprechenden Verbindung der Formel ($I_A$)

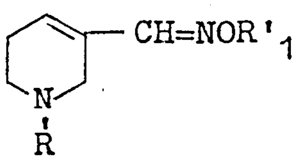

($I_A$)

zu gelangen, die man gegebenenfalls in ein Salz überführt oder die man, wenn $R'_1$ für ein Wasserstoffatom steht, der Einwirkung einer Verbindung der Formel (IV)

$R'_2Hal$ (IV)

unterwirft, worin $R'_2$ einen Rest $COalc_1$ oder einen Rest $-(CH_2)_2N-(alc_2)_2$ bedeutet, wobei $alc_1$ und $alc_2$ wie vorstehend definiert sind und Hal für ein Halogenatom steht, um zu der entsprechenden Verbindung der Formel ($I_B$)

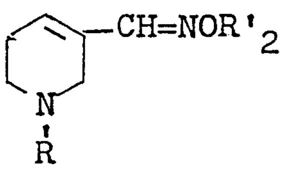

($I_B$)

zu gelangen, die man gegebenenfalls in ein Salz überführt.

**3.** Abänderung des Verfahrens gemäß Anspruch 1, dadurch gekennzeichnet, daß man eine Verbindung der Formel (V)

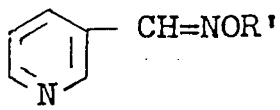

(V)

worin R' die in Anspruch 1 angegebene Bedeutung besitzt, der Einwirkung einer Verbindung der Formel (VI), wie in Anspruch 1 definiert, unterzieht, um zu der Verbindung der Formel (VII)

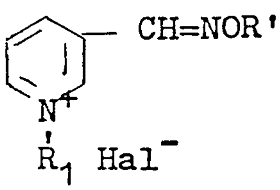

(VII)

zu gelangen, die man der Einwirkung eines Hydrierungsmittels unterzieht, um die entsprechende Verbindung der Formel ($I_C$)

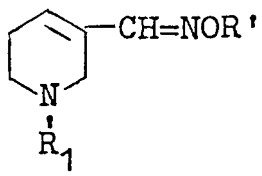

$(I_C)$

zu erhalten, worin $R_1$ und R' die vorstehend angegebene Bedeutung besitzen, die man gegebenenfalls in ein Salz überführt oder, wenn $R_1$ einen durch einen veresterten Carboxyrest substituierten Alkylrest bedeutet, der Einwirkung eines Hydrolysemittels unterzieht, um die entsprechende Verbindung zu erhalten, die eine freie Carboxygruppe aufweist, welche Verbindung man gegebenenfalls in ein Salz überführt.

**4.** Abänderung des Verfahrens gemäß Anspruch 2, dadurch gekennzeichnet, daß man eine Verbindung der Formel (V)

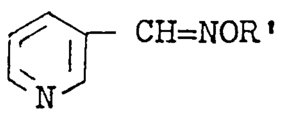

(V)

worin R' die in Anspruch 1 angegebene Bedeutung besitzt, der Einwirkung einer Verbindung der Formel (VI), wie in Anspruch 1 definiert, unterzieht, um zu der Verbindung der Formel (VII)

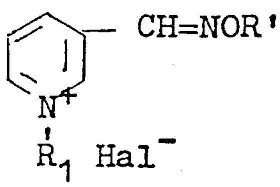

(VII)

zu gelangen, die man der Einwirkung eines Hydrierungsmittels unterzieht, um die entsprechende Verbindung der Formel ($I_C$)

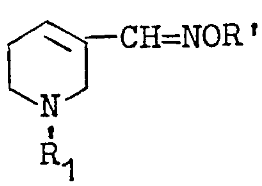

$(I_C)$

zu erhalten, die man gegebenenfalls in ein Salz überführt.

**5.** Verfahren gemäß Anspruch 2, dadurch gekennzeichnet, daß man von einer Verbindung der Formel (II) ausgeht, worin R ein Wasserstoffatom bedeutet.

**6.** Verfahren gemäß Anspruch 2, dadurch gekennzeichnet, daß man von einer Verbindung der Formel (II) ausgeht, worin R einen gesättigten oder ungesättigten Alkylrest mit 1 bis 4 Kohlenstoffatomen bedeutet.

**7.** Verfahren gemäß Anspruch 2, dadurch gekennzeichnet, daß man von einer Verbindung der Formel (II) ausgeht, worin R einen Methyl-, Ethyl-, Propyl- oder Allylrest bedeutet.

**8.** Verfahren gemäß Anspruch 2, dadurch gekennzeichnet, daß man von einer Verbindung der Formel (III) ausgeht, worin $R'_1$ einen Methylrest bedeutet.

**9.** Verfahren gemäß Anspruch 2, dadurch gekennzeichnet, daß man eines der Produkte der Formel ($I_1$) mit den folgenden Bezeichnungen herstellt:

1-Methyl-1,2,5,6-tetrahydropyridin-3-carboxaldehyd-0-methyloxim;
1-Ethyl-1,2,5,6-tetrahydropyridin-3-carboxaldehyd-0-methyl-oxim;
1,2,5,6-Tetrahydropyridin-3-carboxaldehyd-0-methyloxim;
1-Propyl-1,2,5,6-tetrahydropyridin-3-carboxaldehyd-0-methyloxim;
1-Allyl-1,2,5,6-tetrahydropyridin-3-carboxaldehyd-0-methyloxim;

sowie deren Salze und insbesondere deren Hydrochloride.

**10.** Verfahren zur Herstellung von pharmazeutischen Zusammensetzungen, dadurch gekennzeichnet, daß man als Wirkstoff zumindest eines der Produkte der Formel (I), wie in Anspruch 1 definiert, in eine für diese Verwendung bestimmte Form bringt.

**11.** Verfahren zur Herstellung von pharmazeutischen Zusammensetzungen, dadurch gekennzeichnet, daß man als Wirkstoff zumindest eines der Produkte der Formel ($I_1$), wie in Anspruch 2 definiert, in eine für diese Verwendung bestimmte Form bringt.

**12.** Verfahren zur Herstellung von pharmazeutischen Zusammensetzungen, dadurch gekennzeichnet, daß man als Wirkstoff zumindest eines der Produkte der Formel (I), wie in Anspruch 9 definiert, in eine für diese Verwendung bestimmte Form bringt.